# FASCICULE DE BREVET EUROPEEN

(11) **EP 1 663 146 B1**
(45) Date de publication et mention de la délivrance du brevet: **21.06.2017**
(21) Numéro de dépôt: 04816201.0
(22) Date de dépôt: 21.09.2004
(51) Int. Cl.: A61K 9/51

(54) **PROCEDE DE PREPARATION DE MICROPARTICULES BIORESORBABLES, MICROPARTICULES OBTENUES ET UTILISATION**
VERFAHREN ZUR HERSTELLUNG VON BIOLOGISCH RESORBIERBAREN MIKROTEILCHEN, SO ERHALTENE MIKROTEILCHEN UND IHRE ANWENDUNG
METHOD FOR PRODUCTION OF BIORESORBABLE MICROPARTICLES MICROPARTICLES THUS OBTAINED AND USE THEREOF

(30) Priorité: 22.09.2003 FR 0311057
(43) Date de publication de la demande: 07.06.2006
(73) Titulaire: Centre National de la Recherche Scientifique, 75794 Paris Cedex 16 (FR)
(72) Inventeur: ATAMAN-ONAL, Yasemin, F-69003 Lyon (FR); DELAIR, Thierry, F-69700 Echalas (FR); INCHAUSPE, Geneviève, F-69003 Lyon (FR); JEANNIN, Pascale, F-49080 Bouchemaine (FR); PARANHOS-BACCALA, Glaucia, F-69003 Lyon (FR); VERRIER, Bernard, F-69440 Mornant (FR)
(74) Mandataire: Lavoix
(86) Numéro de dépôt international: PCT/FR2004/050447
(87) Numéro de publication internationale: WO 2005/027871

(56) Documents cités:
- WO-A-00/06123
- WO-A-97/02810
- WO-A-98/33487
- WO-A2-03/070909
- NAH J W ET AL: "Clonazepam release from poly(DL-lactide-co-glycolide) nanoparticles prepared by dialysis method." ARCHIVES OF PHARMACAL RESEARCH. AUG 1998, vol. 21, no. 4, août 1998 (1998-08), pages 418-422, XP009031981 ISSN: 0253-6269
- JEON H J ET AL: "Effect of solvent on the preparation of surfactant-free poly(DL-lactide-co-glycolide) nanoparticles and norfloxacin release characteristics." INTERNATIONAL JOURNAL OF PHARMACEUTICS. 10 OCT 2000, vol. 207, no. 1-2, 10 octobre 2000 (2000-10-10), pages 99-108, XP002283839 ISSN: 0378-5173
- MARUYAMA A ET AL: "Nanoparticle DNA carrier with poly(L-lysine) grafted polysaccharide copolymer and poly(D,L-lactic acid).", BIOCONJUGATE CHEMISTRY 1997 SEP-OCT, vol. 8, no. 5, September 1997 (1997-09), pages 735-742, ISSN: 1043-1802
- LAMALLE-BERNARD DELPHINE ET AL: "Coadsorption of HIV-1 p24 and gp120 proteins to surfactant-free anionic PLA nanoparticles preserves antigenicity and immunogenicity.", JOURNAL OF CONTROLLED RELEASE : OFFICIAL JOURNAL OF THE CONTROLLED RELEASE SOCIETY 28 SEP 2006, vol. 115, no. 1, 28 September 2006 (2006-09-28), pages 57-67, ISSN: 0168-3659
- GUILLON CHRISTOPHE ET AL: "Formulation of HIV-1 Tat and p24 antigens by PLA nanoparticles or MF59 impacts the breadth, but not the magnitude, of serum and faecal antibody responses in rabbits.", VACCINE 23 OCT 2007, vol. 25, no. 43, 23 October 2007 (2007-10-23), pages 7491-7501, ISSN: 0264-410X
- PAVOT VINCENT ET AL: "Encapsulation of Nod1 and Nod2 receptor ligands into poly(lactic acid) nanoparticles potentiates their immune properties.", JOURNAL OF CONTROLLED RELEASE : OFFICIAL JOURNAL OF THE CONTROLLED RELEASE SOCIETY 10 APR 2013, vol. 167, no. 1, 10 April 2013 (2013-04-10), pages 60-67, ISSN: 1873-4995
- RANCAN FIORENZA ET AL: "Particle-based transcutaneous administration of HIV-1 p24 protein to human skin explants and targeting of epidermal antigen presenting cells.", JOURNAL OF CONTROLLED RELEASE : OFFICIAL JOURNAL OF THE CONTROLLED RELEASE SOCIETY 28 FEB 2014, vol. 176, 28 February 2014 (2014-02-28), pages 115-122, ISSN: 1873-4995

## Description

La présente invention concerne de nouvelles particules biorésorbables sur lesquelles sont liées des substances protéiques, utiles notamment dans le domaine de la vaccination.

Les substances protéiques, telles que les protéines et les peptides, également appelées antigènes, sont largement utilisées dans le traitement de nombreuses maladies telles que les maladies d'origine virale, généralement sous forme de formulation vaccinale.

Afin d'accroître l'activité, la puissance de l'antigène et d'améliorer la stabilité des compositions pharmaceutiques contenant des antigènes, ces compositions contiennent des adjuvants. A cet égard, les formulations vaccinales comprennent souvent des adjuvants immunologiques pour améliorer les réponses à médiation cellulaire et les réponses humorales.

Malgré la présence de tels adjuvants, les vaccins classiques ont souvent pour inconvénients qu'ils proposent une protection inadéquate contre les agents pathogènes visés. Par protection adéquate, on entend une protection mettant en jeu à la fois la réponse à médiation cellulaire et la réponse humorale du système immunitaire.

Afin d'obtenir une réponse immunitaire adéquate, on a utilisé des supports particulaires associés à des antigènes, lesdits antigènes étant soit adsorbés sur le support, soit piégés dans le support. De tels supports possèdent de multiples copies de l'antigène d'intérêt présenté au système immun et favorisent le piégeage et la rétention des antigènes dans les nodules locaux lymphatiques. Les particules peuvent être phagocytées par des cellules présentatrices d'antigène et peuvent accroître la présentation des antigènes au système immunitaire. Des exemples de tels supports comprennent les polymères de poly(méthacrylate de méthyle), ainsi que les microparticules à base de polylactides, tels que le poly(acide D- ou L-lactique) connu sous le nom de D-PLA ou L-PLA, respectivement, et de poly(lactides-co-glycolides) connus sous le nom de PLG.

Les microparticules à base de PLG dans lesquelles sont piégés des antigènes sont capables de donner une réponse immunitaire. A titre d'exemple, Moore et al. (1995, Vaccine, 13:1741-1749) ont montré que l'antigène gpl20 du virus VIH microencapsulé induisait une réponse T-cellulaire CD4+ et CD8+ VIH-spécifique. De même, Vordermeier et al. (1995, Vaccine, 13-1576-1582) ont montré que l'antigène de Mycobacterium tuberculosis piégé par du PLG induisait chez la souris vaccinée avec un tel antigène tant une réponse humorale qu'une réponse T-cellulaire.

Bien que ces types d'adjuvant offrent quelques avantages par rapport aux autres systèmes plus toxiques, ils ont pour inconvénients que la production de microparticules est difficile et implique l'utilisation de produits chimiques corrosifs, tels que solvants, qui peuvent dénaturer l'antigène et détruire leur immunogénicité. De plus, l'antigène peut également être dégradé du fait de la forte agitation nécessaire lors du procédé de piégeage tel que l'encapsulation.

Il a ainsi été proposé d'utiliser des microparticules en surface desquelles les antigènes sont adsorbés ou greffés (Rock K. L., Efficient MHC I presentation of exogeneous Ag, PNAS 1993). Certains auteurs tels que Eldridge et al. dans Infect. Immun., 59 :2978-2986 (1991) ont toutefois indiqué que, pour atteindre un effet d'adjuvant approprié, les antigènes devaient être piégés dans des microparticules.

La demande de brevet PCT WO97/02810 décrit des particules constituées d'un polymère biodégradable sur lesquelles peuvent être adsorbés des antigènes. Ces particules sont utiles pour la délivrance de ces antigènes. Ces particules ont pour inconvénient leur nature lamellaire, de sorte qu'il n'est pas possible de contrôler leur taille. En effet, les particules destinées à la vaccination doivent posséder une taille submicronique pour être efficaces en transfection et en immunisation.

Les demandes de brevet WO98/33487 et WOOO/06123 décrivent quant à elles des microparticules à base de polylactide ou PLG sur lesquelles sont adsorbés des antigènes et leur utilisation pour stimuler des réponses immunitaires. Toutes les microparticules de ces demandes de brevet, sur lesquelles des antigènes sont adsorbés, ont été obtenues en utilisant un agent tensioactif afin de maintenir la stabilité colloïdale desdites microparticules, ainsi qu'un agent stabilisant, tel que le poly(alcool vinylique), pour la préparation des particules polymères. Les microparticules ainsi obtenues ont pour inconvénient leur toxicité du fait de la présence dans les microparticules dudit agent tensioactif et dudit agent stabilisant.

Il est connu de Nah et al (Archives of Pharmacal Research, 1998) l'encapsulation de protéines dans des particules obtenues par dialyse. Il est également connu de Jeaon et al (international Journal of Phamaceutics, 2000, p.99-108) l'encapsulation de CNZ dans une particule obtenue par dialyse. Enfin, il est connu de WO03/070909 la préparation de particules sans tensioactif ni stabilisant par double émulsion.

La Demanderesse a maintenant découvert contre toute attente que l'on pouvait obtenir des microparticules biorésorbables non lamellaires, de taille au plus micronique, sur lesquelles sont liées des substances protéiques antigéniques, contre lesquelles on cherche à déclencher une réponse immunitaire, et dénuées des inconvénients ci-dessus, à savoir qu'elles ne sont pas toxiques car elles sont préparées sans agent stabilisant et sans agent tensioactif pour la préparation des microparticules polymères, et sans agent tensioactif pour la liaison des antigènes à la surface des microparticules, sans perte de stabilité colloïdale.

Ainsi, un premier objet de l'invention consiste en un procédé de préparation de microparticules biorésorbables sur lesquelles sont liées des substances protéiques, caractérisées en ce qu'il comprend les étapes de :
(i) préparation desdites microparticules par déplacement de solvant à partir d'au moins un polymère biorésorbable choisi parmi poly(acide-α-hydroxylé), poly(acide hydroxybutirique), polycaprolactones, polyorthoesters et polyanhydrides, sans agent stabilisant et sans agent tensioactif, et
(ii) (ii) liaison desdites substances protéiques sur les microparticules obtenues dans l'étape (i) sans agent tensioactif.

Les microparticules obtenues par le procédé de l'invention sont dénuées d'agent stabilisant et d'agent tensioactif de sorte qu'elles sont nouvelles.

Ainsi, un autre objet de l'invention consiste en les microparticules biorésorbables sur lesquelles sont liées des substances protéiques, susceptibles d'être obtenues par le procédé de l'invention.

Contre toute attente, les particules de l'invention conservent leur stabilité colloïdale.

Les microparticules de l'invention sont utiles pour stimuler tant une réponse à médiation cellulaire qu'une réponse humorale, de sorte qu'elles sont utiles tant en thérapeutique qu'en diagnostic.

Ainsi, un autre objet de l'invention consiste en l'utilisation des microparticules de l'invention pour la préparation d'un médicament, ainsi que les compositions pharmaceutiques, notamment vaccins, comprenant les microparticules de l'invention.

Enfin, un autre objet de l'invention consiste en l'utilisation des microparticules de l'invention pour le diagnostic in vitro d'états pathologiques liés à la substance protéique liée sur lesdites microparticules.

Les microparticules destinées à la vaccination ne doivent pas être toxiques pour l'organisme qui les reçoit, tout en conservant leur stabilité colloïdale.

Le procédé de l'invention, n'utilisant ni agent tensioactif, ni agent stabilisant permet contre toute attente l'obtention de telles particules.

Par microparticule, on entend des particules de taille au plus micronique pour leur permettre d'entrer dans les cellules présentatrices d'antigènes.

Par taille au plus micronique, on entend une dimension inférieure ou égale à 999 m. De préférence, les microparticules ont un diamètre de particule inférieure ou égale à 3 m. De façon davantage préférée, les particules de l'invention sont de taille submicronique, avec, de préférence, un diamètre compris entre 150 et 900 nm, de préférence encore entre 250 et 700 nm, de préférence entre 150 et 250 nm.

La taille des particules est facilement déterminée par des techniques connues de l'homme du métier telles que, par exemple, en utilisant la microscopie à balayage électronique, la diffusion quasi-élastique de la lumière, la microscopie électronique à transmission.

Par microparticule toxique, on entend une microparticule comportant au moins un composé susceptible de provoquer des troubles biologiques, tels que des perturbations du métabolisme, dans l'organisme ayant reçu la microparticule.

La première étape du procédé de l'invention consiste en la préparation desdites microparticules à partir d'au moins un polymère biorésorbable sans agent stabilisant et sans agent tensioactif.

Par polymère biorésorbable, on entend un polymère capable de se dégrader, dans l'organisme dans lequel il a été introduit, en composés éliminables par les voies naturelles.

Des exemples de tel polymère biorésorbable comprennent, sans limitation, les poly(acides a-hydroxylés), les poly(acides hydroxybutyriques), les polycaprolactones, les polyorthoesters et les polyanhydrides. De préférence, le polymère biorésorbable utilisé dans le procédé de l'invention est un poly(acide a-hydroxylé) tel que le poly(acide D-lactique), le poly(acide L-lactique) (appelés PLA), le poly(acide glycolique) (appelé PLG), ou bien un mélange de poly(acides a-hydroxylés) tels qu'un mélange de poly(acides D- et L-lactiques), un mélange de poly(acide L-lactique) et de poly(acide glycolique), un mélange de poly(acide D-lactique) et de poly(acide glycolique), ou un mélange de poly(acides D- et L-lactiques) et de poly(acide glycolique), ce qui constitue un mode de réalisation de l'invention.

Lorsque le polymère utilisé dans le procédé de l'invention est un mélange de poly(acides a-hydroxylés), la proportion de chaque constituant peut être facilement déterminée par l'homme du métier. Ainsi, par exemple, on peut utiliser un mélange racémique de poly(acides D- et L-lactiques) ou un mélange PLA/PLG à divers pourcentages connus de l'homme du métier.

Les agents stabilisants habituellement utilisés dans les procédés de préparation de microparticules comprennent par exemple le poly(alcool vinylique), les Pluronics (copolymère de poly(oxyde d'éthylène) et de poly(oxyde de propylène), des tensioactifs cationiques ou anioniques tels que le bromure de cétyltriméthylammonium ou le sel de sodium du sulfate de dodécyle.

Bien entendu, quand l'agent stabilisant utilisé dans les procédés de l'art antérieur est un agent tensioactif, lesdits procédés n'utilisent pas d'agent tensioactif supplémentaire.

Les agents tensioactifs habituellement utilisés dans les procédés de l'art antérieur sont largement connus de l'homme du métier et sont décrits par exemple dans la demande de brevet WO98/33487.

La deuxième étape du procédé de l'invention consiste à procéder à la liaison de substances protéiques sur les microparticules obtenues dans la première étape du procédé, sans utiliser d'agent tensioactif.

Cette étape de liaison de la substance protéique à la surface des microparticules a pour caractéristique qu'elle est effectuée sans agent tensioactif. En effet, contre toute attente, même en l'absence d'agent tensioactif, les microparticules de l'invention présentent une stabilité colloïdale assurant un domaine de taille de particules adaptée à l'utilisation en immunisation.

A titre d'exemples d'agent tensioactif habituellement utilisé pour la liaison de substances protéiques à la surface de microparticules, on pourra se référer aux tensioactifs cités précédemment.

Les substances protéiques à lier à la surface des microparticules obtenues dans l'étape (i) du procédé de l'invention peuvent être toute substance protéique contre lesquelles on cherche à déclencher une réponse immunitaire.

Par réponse immunitaire, on entend une réponse à médiation cellulaire, une réponse humorale ou les deux.

Par réponse à médiation cellulaire, on entend une réponse médiée par les lymphocytes T et/ou d'autres leucocytes. Cette réponse se traduit par l'induction d'une activité lytique par les lymphocytes T cytotoxiques et/ou par la production de cytokine par les lymphocytes T CD8+ suppressifs ou par les cellules T auxiliaires (helper).

Par réponse humorale, on entend une réponse médiée par les molécules anticorps sécrétés par les lymphocytes B.

Les substances protéiques appropriées aux fins de l'invention peuvent être de plusieurs origines telles que d'origine virale ou bactérienne.

A titre d'exemple de telles substances protéiques, on peut citer par exemple les antigènes et les épitopes ou toute substance protéique ayant un rôle d'antigène après liaison aux microparticules.

Les antigènes sont des molécules susceptibles d'être reconnues par un anticorps dont ils ont induit la synthèse par une réponse immunitaire et contenant au moins un épitope. Ce peut être des protéines entières ou des fragments de protéine ayant conservé la structure d'intérêt.

Les épitopes sont des peptides comprenant entre 3 et 15 et généralement entre 5 et 15 acides aminés, ayant également conservé la structure d'intérêt.

Selon un mode de réalisation particulier de l'invention, la substance protéique est un antigène d'origine virale.

Lorsque la substance protéique est d'origine virale, les virus appropriés sont tous virus pour lesquels on connaît des substances capables d'une réponse immunitaire.

A titre d'exemple, on peut citer, sans aucune limitation, les herpes virus, les virus des hépatites tels que le virus de l'hépatite B (VHB) ou le virus de l'hépatite C (VHC), les papillomas virus (HPV) et les virus de l'immunodéficience humaine (VIH), tels que VIH-1 et VIH-2.

Les séquences nucléiques des virus appropriés aux fins de l'invention, ainsi que les protéines codées par lesdites séquences sont largement connues de l'homme du métier et sont disponibles par exemple dans des bases de données telles que GenBank.

Ainsi, par exemple, le virus VIH a des gènes qui codent pour des protéines structurelles du virus. Le gène *gag* code pour la protéine qui forme le core du virion, incluant l'antigène p24. Le gène *pol* code pour les enzymes responsables de la transcription inverse (transcriptase inverse), du clivage (protéase) et de l'intégration (intégrase). Le gène *env* code pour les glycoprotéines d'enveloppe. Il contient six autres gènes (*tat, rev, nef, vif, vpr* et *vpu* (VIH-1) ou *vpx* (VIH-2)) qui codent pour des protéines impliquées dans la régulation de l'expression des gènes du virus (protéines de régulation). Le génome du VIH comprend également les LTR 5' et 3' (Long Terminal Repeat) qui comprennent des éléments de régulation impliqués dans l'expression des gènes du virus.

Selon un mode de réalisation de l'invention, la substance protéique utilisée dans le procédé de l'invention est un antigène du virus VIH. De préférence, l'antigène du virus VIH est une protéine de régulation ou la protéine p24, les protéines de régulation préférées étant la protéine Tat, Rev ou Nef.

S'agissant du VHC, l'extrémité 5' de son génome correspond à une région non traduite adjacente aux gènes qui codent pour les protéines structurales, la protéine core de la nucléocapside, les deux glycoprotéines d'enveloppe, E1 et E2, et une petite protéine appelée p7. La région non traduite 5' et le gène core sont relativement bien conservés dans les différents génotypes. Les protéines d'enveloppe E1 et E2 sont codées par des régions plus variables d'un isolat à un autre. La protéine p7 est une protéine extrêmement hydrophobe qui constituerait un canal ionique. L'extrémité 3' du génome du VHC contient les gènes qui codent pour les protéines non structurales (NS2, NS3, NS4, NS5) et pour une région 3' non codante possédant un domaine bien conservé (Major ME, Feinstone SM, Hepatology, juin 1997, 25(6) : 1527-1538).

La protéine non structurale NS3 du VHC est une protéine de 630 acides aminés qui comprend deux domaines structuraux distincts : un domaine N-terminal, de 81 acides aminés, doté d'une activité protéasique à sérine active intervenant dans la maturation de la protéine virale (domaine appelé NS3 protéase), et un domaine C-terminal, de 549 acides aminés, comprenant une activité hélicase associée à une activité NTPasique qui joue un rôle dans la réplication du génome viral (domaine appelé NS3 hélicase). Cette protéine NS3 est relativement bien conservée parmi les différents génotypes du virus, de sorte que cette protéine constitue un antigène « candidat vaccin » de choix.

Selon un mode de réalisation de l'invention, la substance protéique d'intérêt est une protéine antigénique du VHC, de préférence une protéine non structurale, de préférence encore la protéine NS3 et en particulier le protéine NS3 hélicase étant davantage préférées.

Les substances protéiques appropriées aux fins de l'invention peuvent être obtenues par la technique du génie génétique qui comprend les étapes de :
- culture d'un microorganisme ou de cellules eucaryotes transformé(es) à l'aide d'une séquence nucléotidique codant pour la substance protéique d'intérêt et
- récupération de ladite substance protéique produite par ledit microorganisme ou lesdites cellules eucaryotes.

Cette technique est bien connue de l'homme du métier. Pour plus de détail la concernant, on pourra se référer à l'ouvrage ci-après : Recombinant DNA Technology I, Editors Ales Prokop, Raskesh K Bajpai ; Annals of the New-York Academy of Sciences, Volume 646, 1991.

Les substances protéiques d'intérêt, lorsqu'elles sont de petites tailles, peuvent également être préparées par les synthèses peptidiques classiques bien connues de l'homme du métier.

La liaison des substances protéiques sur les microparticules biorésorbables peut être mise en oeuvre par tout procédé connu de l'homme du métier.

Des exemples de telle liaison comprennent l'adsorption, la liaison covalente et la liaison par l'intermédiaire d'un polymère polysaccharidique déposé en surface de la microparticule, tel qu'un chitosane, la substance protéique étant liée au chitosane par adsorption.

L'adsorption peut être mise en oeuvre par exemple en mélangeant les microparticules avec les substances protéiques et en incubant sous agitation, par exemple à température ambiante ou à 37°C.

La liaison covalente des substances protéiques à la surface des microparticules peut être mise en oeuvre en utilisant les techniques et réactifs connus dans la littérature, comme par exemple décrit dans Bioconjugate Techniques, G. T. Hermanson, Academic Press, London, 1996 et Chemical Reagents for Protein Modification, R. L. Lundblad, Ed. CRC Press, 1991.

Selon un mode de réalisation particulier, la liaison des substances protéiques sur les microparticules est mise en oeuvre par adsorption.

Les microparticules biorésorbables sur lesquelles sont liées de substances protéiques, préparées selon le procédé de l'invention, sont dénuées d'agent stabilisant et d'agent tensioactif, de sorte qu'elles sont nouvelles et constituent un autre objet de l'invention.

Les microparticules de l'invention, du fait de leur capacité à induire une réponse immunitaire grâce à la substance protéique, et du fait de leur absence de toxicité, sont particulièrement bien adaptées pour la préparation de compositions pharmaceutiques, notamment vaccins, utiles dans le traitement de pathologies associées à la substance protéique liée aux microparticules.

Ainsi, un autre objet de l'invention consiste en l'utilisation des microparticules biorésorbables de l'invention pour la préparation d'un médicament

En particulier, le médicament préparé avec les microparticules de l'invention est particulièrement utile pour l'inhibition, la prévention ou le traitement d'une infection provoquée par un virus, tel que par exemple le virus VIH ou VHC ou tout autre virus connu, ce qui constitue un autre mode de réalisation de l'invention.

L'invention concerne également une composition pharmaceutique, notamment vaccin, contenant au moins une microparticule de l'invention et le cas échéant un excipient pharmaceutiquement acceptable.

Les compositions pharmaceutiques de l'invention sont appropriées pour l'administration orale, sublinguale, sous-cutanée, intramusculaire, intraveineuse, topique, locale, intratrachéale, intranasale, transdermique, rectale, intraoculaire, intra-auriculaire, ledit principe actif pouvant être administré sous forme unitaire d'administration.

Les formes unitaires d'administration peuvent être par exemple des comprimés, des gélules, des granules, des poudres, des solutions ou suspensions orales injectables, des timbres transdermiques («patch»), des formes d'administration sublinguale, buccale, intratrachéale, intraoculaire, intranasale, intra-auriculaire, par inhalation, des formes d'administration topique, transdermique, sous-cutanée, intramusculaire ou intraveineuse, des formes d'administration rectale ou des implants. Pour l'administration topique, on peut envisager des crèmes, gels, pommades, lotions ou collyres.

Ces formes galéniques sont préparées selon les méthodes usuelles des domaines considérés.

Bien entendu, l'homme du métier déterminera facilement l'excipient approprié et la quantité de microparticules à utiliser en fonction des constituants et de la forme unitaire d'administration de la composition pharmaceutique.

Lesdites formes unitaires sont dosées pour permettre une administration journalière de 0,001 à 10 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse du patient.

Selon un autre mode de réalisation de l'invention, la présente invention concerne également une méthode de traitement des pathologies associées à la substance protéique liée à la microparticule de l'invention, qui comprend l'administration, à un patient, d'une dose efficace d'une composition pharmaceutique de l'invention.

Les microparticules de l'invention permettent également d'obtenir des anticorps, ce qui constitue un autre objet de l'invention.

Les anticorps selon l'invention sont des anticorps polyclonaux ou monoclonaux, les anticorps monoclonaux étant préférés.

Les anticorps polyclonaux sus-mentionnés peuvent être obtenus par immunisation d'un animal avec au moins une microparticule de l'invention, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment celui de la microparticule de l'invention.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec au moins une microparticule de l'invention, dont les lymphocytes B sont alors capables de produire des anticorps contre la substance protéique de ladite microparticule. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la microparticule de l'invention pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les microparticules, ainsi que les anticorps de l'invention sont également utiles dans le diagnostic de l'état pathologique associé à la substance protéique liée à la surface de ladite microparticule.

En effet, les microparticules ou anticorps de l'invention, peuvent être utilisés en tant que partenaire de capture ou de détection d'analyte dans toute technique de diagnostique utilisant de tels partenaires, telle que la méthode ELISA. Par exemple, lorsqu'on veut rechercher un antigène à titre d'analyte, on utilise un anticorps de l'invention obtenu à partir de microparticules sur lesquelles sont liées ledit antigène, tandis que si on veut chercher des anticorps, on utilise les microparticules de l'invention. Dans ce dernier cas, si le test diagnostic nécessite l'utilisation d'un support solide, les microparticules peuvent ou non jouer ce rôle.

Ainsi, un autre objet de l'invention consiste en une composition diagnostique constituée des microparticules biorésorbables ou des anticorps de l'invention.

Elle concerne également l'utilisation de cette composition diagnostique pour le diagnostic *in vitro* de l'état pathologique lié à la substance protéique liée à la microparticule biorésorbable, l'état pathologique pouvant être, selon un mode de réalisation, une infection virale, telle que provoquée par le virus VIH ou le virus VHC.

Là encore, l'homme du métier déterminera facilement la quantité de microparticules ou anticorps à utiliser en fonction de la technique diagnostique utilisée.

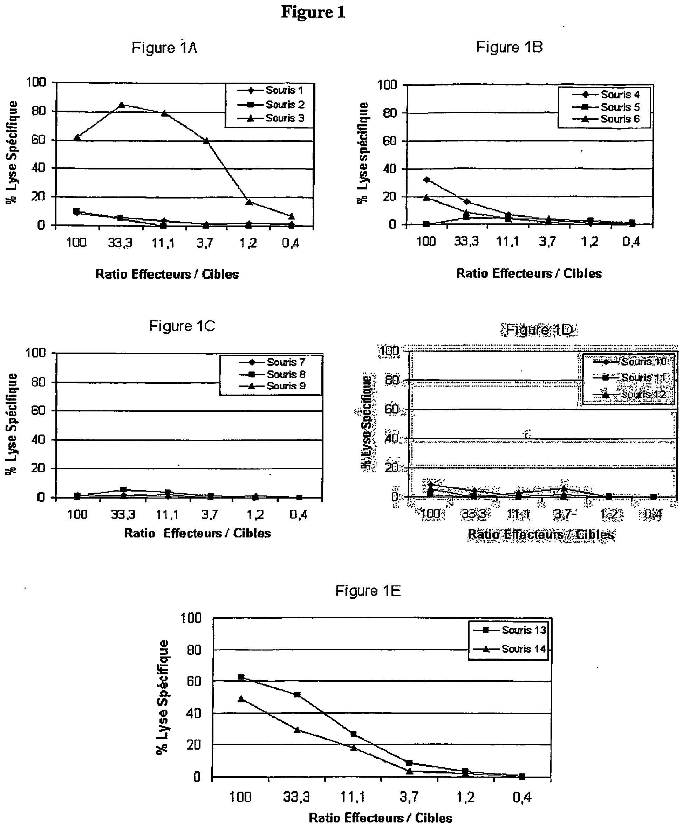

La présente invention sera mieux comprise à l'aide des exemples suivants donnés uniquement à titre illustratif et non limitatif, ainsi qu'à l'aide des figures 1 à 9, dans lesquelles :
- la figure 1 donnant le résultat de tests CTL en immunisant des souris avec la séquence d'ADN correspondant à la polyprotéine NS3NS4 du VHC à titre de témoin (figure 1A), avec la substance protéique NS3 hélicase et l'adjuvant de Freund, sans microparticule (figure 1B), avec la substance protéique NS3 hélicase sans microparticule (figure 1C), avec des microparticules de PLA sans substance protéique (figure 1D), et avec les microparticules de l'invention dans lesquelles le polymère est le PLA et la substance protéique est le peptide NS3 hélicase (figure 1E),

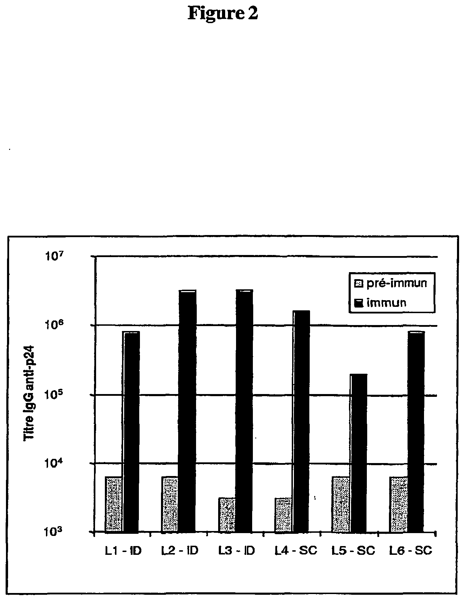

- la figure 2 représente un histogramme donnant les titres en IgG anti-p24 selon un test ELISA, dans les sérums préimmuns et les sérums immuns de 6 lapins (L1 à L6) ayant reçu une injection des particules de l'invention PLA-p24 soit par voie intradermique (ID), soit par voie sous-cutanée (SC),

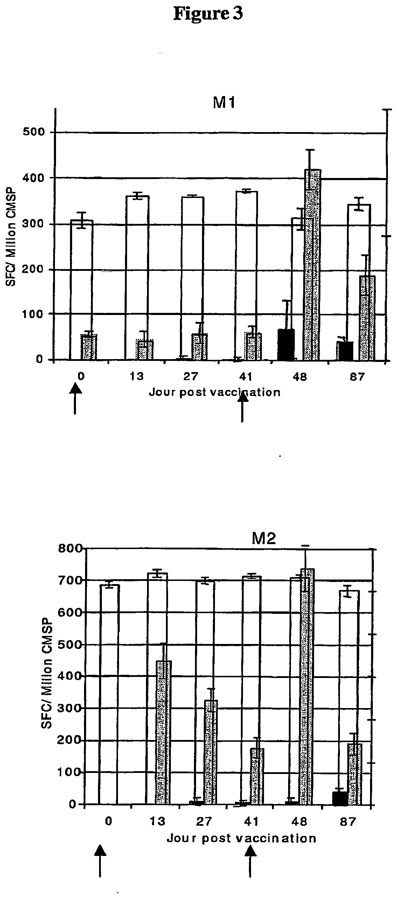

- la figure 3 représente des histogrammes (moyenne de 4 réplicats +/- écarts type) obtenus selon un test ELISPOT chez 2 macaques M1 et M2 ayant reçu des microparticules de l'invention PLA/p24, donnant le nombre de spots par millions de cellules obtenus en fonction des jours post vaccination après stimulation sans antigène (milieu, témoin négatif, histogramme noir), après stimulation avec la p24 (histogramme gris) ou après stimulation par le couple PMA Ionomycine (histogramme contour),

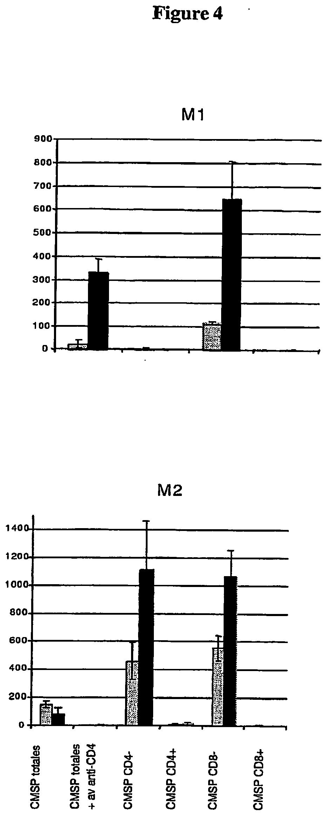

- la figure 4 représente des histogrammes donnant le nombre de spots obtenus en ELISPOT chez 2 macaques M1 et M2 ayant reçu des microparticules de l'invention PLA/p24, après stimulation soit avec la protéine p24 (histogramme noir), soit avec les peptides (histogramme gris), dans la fraction de CMSP totales, la fraction de CMSP totales en présence de l'anticorps anti-CD4, la fraction de CMSP déplétée en CD4⁺ (CMSP CD4-) et la fraction enrichie en CD4⁺ correspondante (CMSP CD4+), ainsi que la fraction de CMSP déplétée en CD8⁺ (CMSP CD8-) et la fraction enrichie en CD8⁺ correspondante (CMSP CD8+),

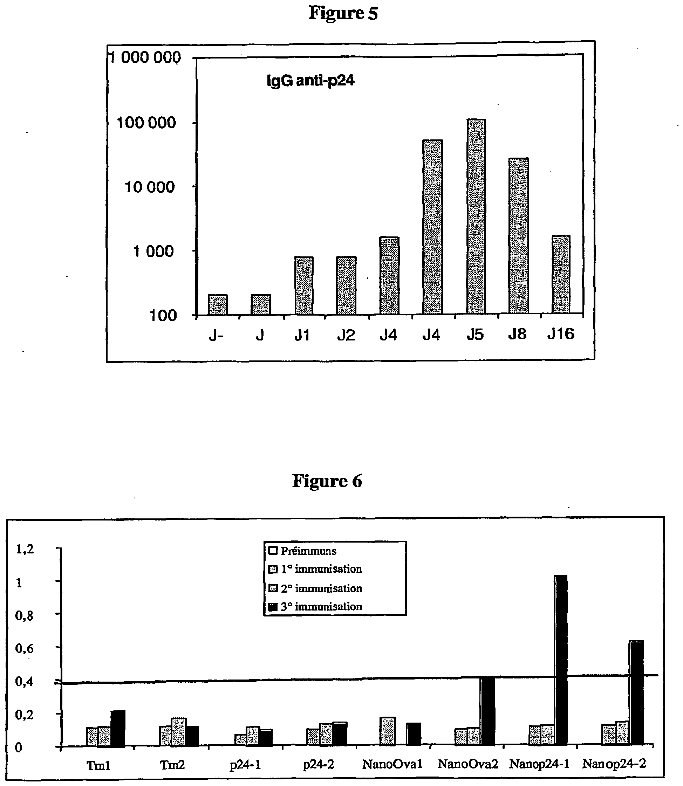

- la figure 5 représente des histogrammes donnant le titre en IgG anti-p24 obtenus par ELISA chez le singe M2 ayant reçu des microparticules de l'invention PLA/p24, en fonction des jours de prélèvement des sérums séquentiels,
- la figure 6 représente des histogrammes donnant la valeur de DO obtenue en ELISA dans le sérum préimmun, dans le sérum après la 1^{ère} immunisation, dans le sérum après la deuxième immunisation et dans le sérum après la 3^{ème} immunisation de souris immunisées par des cellules SRDC et sensibilisées soit par le témoin négatif (Tm1 et Tm2), soit par la protéine p24 (p24-1 et p24-2), soit par le témoin négatif microparticules (NanoOval et NanoOva2) et soit par les microparticules de l'invention (Nanop24-1 et Nanop24-2),

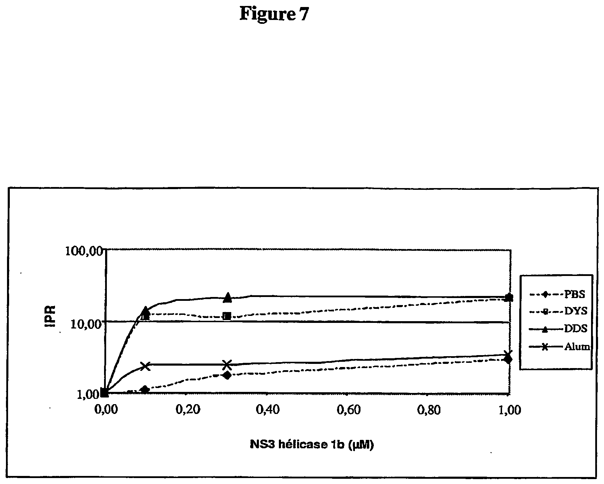

- la figure 7 représente des graphes donnant l'indice de prolifération relative (IPR) spécifique à la protéine NS3h en fonction de la quantité de NS3h utilisée chez des souris ayant reçu du NS3h-PBS (PBS), des microparticules de l'invention préparée par dialyse (DYS), des microparticules de l'invention préparée par déplacement de solvant (DDS) et la composition NS3h-Alum,

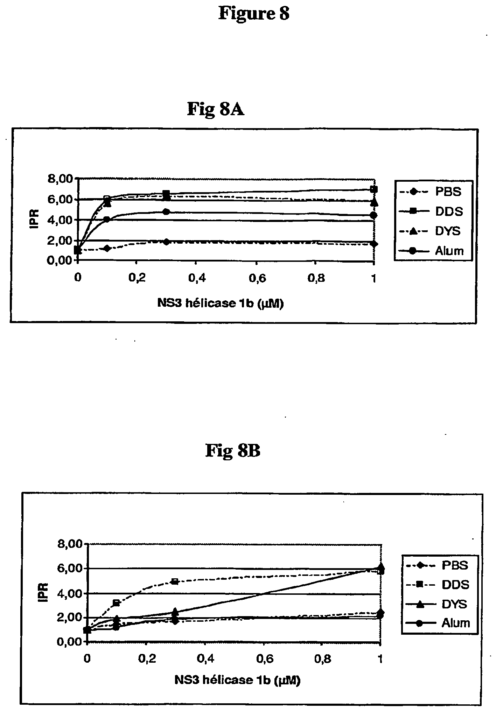

- la figure 8 représente des graphes donnant l'indice de prolifération relative (IPR) spécifique à la protéine NS3h en fonction de la quantité de NS3h utilisée chez des souris ayant reçu du NS3h-PBS (PBS), des microparticules de l'invention préparée par dialyse (DYS), des microparticules de l'invention préparée par déplacement de solvant (DDS) et la composition NS3h-Alum (Alum) dans les cellules des ganglions poplités pour la réponse cellulaire localisée (figure 8A) et dans les cellules de la rate pour la réponse cellulaire systémique (figure 8B), et

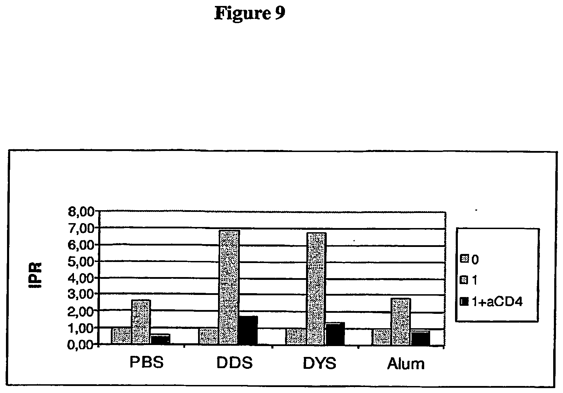

- la figure 9 représente des histogrammes donnant l'indice de prolifération cellulaire (IPR) en fonction des immunogènes utilisés chez des souris, à savoir du NS3h-PBS (PBS), des microparticules de l'invention préparées par dialyse (DYS), des microparticules de l'invention préparées par déplacement de solvant (DDS) et la composition NS3h-Alu (Alum), dans les cellules de la rate sans stimulation (0), après stimulation avec de la protéine NS3h (1) ou après stimulation avec de la protéine et de l'anticorps anti-CD4⁺ (1+aCD4).

### Exemple 1 : Préparation de microparticules de l'invention par dialyse

### 1. Préparation de particules de PLA

On a utilisé du PLA 50 (50% de poly(acide L-lactique) et 50% de poly(acide D lactique)) de masse molaire 52000 Da (Phusis®).

On a dissous ce PLA à 2% en poids total de solution dans du DMSO (Prolabo®). On a ensuite introduit la solution organique de PLA dans un boudin de dialyse de Cut Off 15000 Da (Spectrum®) et on a placé l'ensemble dans un bain-marie d'eau bidistillée (41, MilliQ®) agité et changé régulièrement toutes les heures, pendant 6h. On a poursuivi le dernier bain de dialyse toute la nuit pour obtenir des particules en précipité.

Le lendemain, on a récupéré la solution de particules de PLA et on l'a stockée à 4°C.

On a caractérisé les particules ainsi obtenues par leur taille, leur indice de polydispersité et leur charge à l'aide de l'appareil Zetasizer 3000 HS (Malvern® Instruments). On a également évalué leur taux de solide après pesée par le calcul : (Masse extrait sec/Masse extrait humide)×100.

### 2. Préparation de particules de PLA/p24

On a préparé la protéine p24 du VIH-1 sous forme recombinante dans E. coli et on l'a purifiée par chromatographie par affinité métal-chélate selon la technique de Cheynet V., et al., 1993, Protein Expr. Purif., 4 :367-372.

Les microparticules de PLA ont été préparées comme décrit dans le point 1 ci-dessus et ont un diamètre de particule de 515,7 +/- 6,7 nm, un taux de solide de 1,1% et un indice de polydispersité de 0,242 +/- 0,013.

On a préparé un tampon phosphate 10mM, pH 5,7 en mélangeant 10 ml de tampon phosphate 0,1M, pH 4,7 (NaH₂PO₄, 2 H₂O, M=15,60g/l) et 1,1 ml de tampon phosphate 0,1M, pH 9,2 (NaH₂PO₄, 2 H₂O, M=17,79g/l) et on a dilué au 1/10^{è} avec de l'eau.

On a mélangé 200 µl de la protéine p24 diluée à 0,6 g/l dans le tampon phosphate 10mM, pH 5,7 et 200 µl des microparticules, et on a agité toute la nuit sur roue à température ambiante. On a ensuite centrifugé 5 min à 5000 tpm et on a vidé le surnageant qui a permis de doser la quantité de p24 non adsorbée (kit BCA Protein Assay de PIERCE) et d'en déduire la concentration de p24 adsorbée sur les microparticules qui s'élève à 0,2 g/l.

### 3. Préparation des microparticules de PLA/Tat

On a utilisé la protéine Tat du VIH-1, de séquence SEQ ID N°1, synthétisée selon le mode opératoire décrit dans Péloponèse J.P., et al., 1999, The Journal of Biological Chemistry, 274(17) : 11473-11478.

Les microparticules de PLA ont été préparées comme décrit dans le point 1 ci-dessus et ont un diamètre de particule de 420,1 +/- 10,7 nm, un taux de solide de 1,02% et un indice de polydispersité de 0,241 +/- 0,040.

On a mélangé 200 µl de la protéine Tat diluée à 0,4 g/l dans un tampon phosphate 10mM, pH 6,8 dégazé, préparé comme indiqué dans le point 2 ci-dessus, à ceci près qu'on a utilisé 13,8 ml de tampon phosphate 0,1M, pH 9,2, et 200 µl des microparticules, et on a agité toute la nuit sur roue à température ambiante. On a ensuite centrifugé 5 min à 5000 tpm et on a vidé le surnageant qui a permis de doser la quantité de Tat non adsorbée (kit BCA Protein Assay de PIERCE) et d'en déduire la concentration de Tat adsorbée sur les microparticules qui s'élève à 0,1 g/l.

### 4. Préparation des microparticules de PLA/NS3 hélicase

On a utilisé le peptide NS3 hélicase de séquence SEQ ID N°2 du VHC obtenu sous forme recombinante comme suit.

Le gène codant pour les acides aminés 1192-1458 correspondant au domaine hélicase de la protéine NS3 du VHC en fusion avec l'hexahistidine a été cloné dans le vecteur d'expression procaryote pMH80 et exprimé dans les bactéries E. coli JM109 (Promega). L'expression de la protéine recombinante a été réalisée à 30°C après 3 heures d'induction par lTPTG 1mM (isopropyl-beta-D-thiogalactopyranoside, Promega). Après centrifugation, les bactéries ont été lysées par sonication dans la solution tampon: Tris-HCL 10mM, pH8, MgCl2 5mM, Triton X100 1%, anti-protéase 1 pastille (Boehringer) bensonase 250U (Merck). Après lyse et centrifugation, la fraction soluble a été purifiée sur colonne de Niagarose et éluée dans la solution tampon phosphate de sodium 20mM pH7,2, NaCl 300mM et imidazole 300mM. La protéine pure a été ainsi dialysée contre du PBS pH 7,2. Après purification, la protéine a été analysée en électrophorèse en gel d'acrylamide en présence de dodécylsulfate de sodium (SDS), spectrométrie de masse. Le degré de pureté de la protéine NS3, domaine helicase, est estimé à supérieur 95%. On a vérifié l'absence d'endotoxine en mesurant le dégré d'endotoxines (LPS) par un test LAL et fonctionnel *in vitro.*

Les microparticules de PLA ont été préparées comme décrit dans le point 1 ci-dessus et ont un diamètre de particule de l'ordre de 600 nm, un taux de solide de 1% et un indice de polydispersité de 0,2.

On a mélangé 200 µl du peptide NS3 hélicase dilué à 0,327 g/l dans un tampon phosphate 10mM, pH 6,5, préparé comme décrit dans le point 2 ci-dessus, à ceci près qu'on a utilisé 6,8 ml de tampon phosphate 0,1M, pH 9,2, et 200 µl des microparticules, et on a agité toute la nuit sur roue à température ambiante. On a ensuite centrifugé 5 min à 5000 tpm et on a vidé le surnageant qui a permis de doser la quantité de NS3 hélicase non adsorbée (kit BCA Protein Assay de PIERCE) et d'en déduire la concentration de NS3 hélicase adsorbée sur les microparticules qui s'élève à 0,28 g/l.

### Exemple 2 : Préparation de microparticules de l'invention par déplacement de solvant

### 1. Préparation de particules de PLA

On a utilisé du PLA 50 (50% de poly(acide L-lactique) et 50% de poly(acide D lactique)) de masse molaire 52000 Da (Phusis®).

On a dissous ce PLA à 2% en poids total de solution dans de l'acétone. On a ensuite ajouté goutte à goutte la solution de PLA dans l'acétone à 35 ml d'eau et on a évaporé le solvant sous pression réduite pendant 35 min.

On a caractérisé les particules ainsi obtenues par leur taille, leur indice de polydispersité et leur charge à l'aide de l'appareil Zetasizer 3000 HS (Malvern® Instruments). On a également évalué leur taux de solide après pesée par le calcul : (Masse extrait sec/Masse extrait humide)×100.

### 2. Préparation des microparticules de PLA/NS3 hélicase

On a utilisé le peptide NS3 hélicase de séquence SEQ ID N°2 du VHC obtenu sous forme recombinante comme indiqué dans l'exemple 1, point 4 ci-dessus.

Les microparticules de PLA ont été préparées comme décrit dans le point 1 ci-dessus et ont un diamètre de particule de l'ordre de 250 nm, un taux de solide de 1% et un indice de polydispersité de 0,3.

On a mélangé 200 µl du peptide NS3 hélicase dilué à 0,327 g/l dans un tampon PBS (tampon Phosphate Buffer Saline ; NaCl 150mM, pH 7,1), préparé comme décrit dans le point 1 ci-dessus, à ceci près qu'on a utilisé 6,8 ml de tampon PBS 0,1M, pH 9,2, et 200 µl des microparticules, et on a agité toute la nuit sur roue à température ambiante. On a ensuite centrifugé 5 min à 5000 tpm et on a vidé le surnageant qui a permis de doser la quantité de NS3 hélicase non adsorbée (kit BCA Protein Assay de PIERCE) et d'en déduire la concentration de NS3 hélicase adsorbée sur les microparticules qui s'élève à 0,34 g/l.

### Exemple 3 : Immunisation de souris avec les microparticules de l'invention PLA/p24

### 1. Modèle animal

Les expériences d'immunisation ont été réalisées chez des souris BALB/c (H-2^{d}) femelles âgées de 6 à 8 semaines au moment de la première immunisation.

### 2. Immunogènes administrés

Dans cette expérience, on a utilisé la protéine p24 seule, les microparticules de l'invention PLA/p24 préparées comme indiqué dans l'exemple 1, point 2 ci-dessus, ainsi que la composition p24-adjuvant de Freund (Sigma) préparée sous forme d'émulsion eau-dans-huile et dont il est connu qu'elle présente un bon pouvoir immunogène (témoin positif).

### 3. Immunisations

Les souris ont reçu 3 doses successives (40 µg ou 10 µg chacune) des immunogènes décrits dans le point 2 ci-dessus à 0, 2 et 4 semaines. Toutes les injections ont été réalisées par voie sous-cutanée.

Les animaux ont été sacrifiés 10 jours (J38), 14 jours (J42) ou bien 42 jours (J70) après la troisième injection et le sang et la rate ont été prélevés pour les analyses immunologiques

### 4. Analyses immunologiques

On a recherché la réponse humorale et la réponse cellulaire comme suit :
- Réponse humorale : on a effectué un prélèvement de sang sur les souris avant leur sacrifice. La présence des anticorps anti-p24 (IgG1, IgG2a et IgG) a été déterminée par ELISA. La protéine p24 a été utilisée en capture et les anticorps spécifiques présents dans le sérum ont été révélés par des anticorps polyclonaux anti-souris à titre d'anticorps de détection, lesquels se lient aux anticorps recherchés et sont respectivement un anticorps de chèvre anti-IgG1 de souris marqué à la peroxydase de raifort (Southern Biotechnology Associates Inc., Cat no 1070-05, Birmingham, Alabama, USA), un anticorps de chèvre anti-IgG2a de souris marqué à la peroxydase de raifort (Southem Biotechnology Associates Inc., Cat no 1080-05), et un anticorps de chèvre anti-IgG de souris AffiniPure conjugué à la peroxydase de raifort (H+L, Jackson Immunoresearch, Cat no 115-035-062). Le titre est l'inverse de la dilution pour laquelle on obtient une absorbance de 0,3 unité DO avec le protocole ELISA utilisé. Le ratio des isotypes IgG2a : IgG1 qui permet de juger de la tendance IFN-γ - IL-4 (respectivement Th1 - Th2) de la réponse immune a aussi été déterminé par ELISA indirect
- Réponse cellulaire : après sacrifice des souris, les rates ont été prélevées de façon stérile pour préparer une suspension cellulaire. Les analyses suivantes ont été pratiquées sur les suspensions cellulaires obtenues, chaque souris ayant été analysée de manière individuelle
   *(i) Test CTL :* La suspension cellulaire a été mise en culture en présence d'un peptide de 9-mer (AMQMLKETI, SEQ ID N°3) qui correspond à un épitope CTL *H-2K^{d}*-restreint immunodominant, et d'IL-2. Cinq jours plus tard, la population effectrice a été restimulée par des cellules naïves irradiées, chargées avec le peptide. La population cytotoxique effectrice a été récoltée au bout du 7^{ème} jour et l'activité CTL a été mesurée en utilisant comme cibles les cellules P815 marquées au ⁵¹Cr.
   *(ii) ELISPOT* : L'ELISPOT permet de déterminer le nombre de cellules sécrétant une cytokine donnée en réponse à un stimulus spécifique. Nous nous sommes intéressés à la cytokine IFN-γ (Th1). Les suspensions cellulaires obtenues à partir des rates ont été restimulées *in vitro* par le peptide AMQMLKETI pendant 20h pour analyser les réponses de type CD8.

Les plaques d'ELISPOT 96 puits avec des membranes de PVDF (Multiscreen IP, Millipore) ont été coatées par un anticorps anti-IFN-γ. Pendant la restimulation, les suspensions de splénocytes ont été incubées dans ces plaques de manière à capturer les cytokines sécrétées par chaque cellule. Les spots correspondant à chaque cellule sécrétant la cytokine d'intérêt ont été révélés par un anticorps de détection biotinylé spécifique de la cytokine d'intérêt.
(iii) *Prolifération* : Les splénocytes ont été stimulées en présence de la protéine p24 pendant 5 jours. Les cellules ont été pulsées pendant 18h par de la thymidine tritiée, qui s'incorpore dans l'ADN des cellules qui sont en train de proliférer. A la suite du pulse, les cellules ont été récoltées sur une membrane qui retient l'ADN et permet d'éliminer par lavage la thymidine marquée non incorporée. Plus les cellules prolifèrent en réponse au stimulus spécifique, plus l'ADN est marqué ; en d'autres termes, plus la réponse cellulaire contre l'immunogène (p24) est importante.

### 5. Résultats

Une première série d'expérience a été réalisée avec 15 souris (5 souris par bras), trois doses de 10 µg d'immunogène et un sacrifice des souris à J38, et en recherchant la réponse humorale et le test CTL et la prolifération à titre de réponse cellulaire.

Les résultats sont indiqués dans le tableau 1 ci après :

**Tableau 1**

| | p24 seule | p24/Freund | p24/PLA |
|---|---|---|---|
| Prolifération (Δcpm)^{a} | 3000 | 3900 | 8000 |
| CTL^{b} | 0/5 | 0/5 | 0/5 |
| Anticorps IgG1 (titre)^{c} | 0,1x10⁵ | 10x10⁵ | 7x10⁵ |

| | | | |
|---|---|---|---|
| ^{a} Moyenne des valeurs cpm (stimulation spécifique - stimulation milieu), cpm = coups par minute (Test de Student, P=0,002) ^{b} Nombre de souris ayant une activité CTL spécifique sur le nombre de souris total du bras ^{c} Moyenne géométrique des titres IgG1 anti-p24 des souris du bras | | | |

Ce tableau met en évidence que :
- on obtient des réponses prolifératives améliorées avec les microparticules de l'invention p24/PLA, par rapport à la protéine p24 seule ou adjuvantée par du Freund,
- aucune activité CTL n'est détectée avec l'un quelconque des immunogènes, et
- les microparticules de l'invention p24/PLA permettent d'obtenir un titre en anticorps spécifique largement supérieur à celui obtenu lors de l'administration de p24 seule, les réponses obtenues étant dans l'ordre de grandeur des titres en anticorps obtenus avec la combinaison p24/adjuvant de Freund.

On a répété l'expérimentation avec 13 souris (3 ou 4 souris par bras), à ceci près qu'on a utilisé les immunogènes à raison de 40 µg et que le sacrifice des souris a été effectué soit à J42 (3 souris), soit à J70 (4 souris).

Les résultats sont indiqués dans le tableau 2 ci-dessous.

**Tableau 2**

| | p24 seule | p24/Freund | p24/PLA | p24/PLA |
|---|---|---|---|---|
| | | | J42 | J70 |
| ELISPOT IFN-γ CD8^{a} | 6 | 100 | 50 | 330 |
| CTL^{b} | 0/3 | 0/3 | 0/3 | 4/4 |
| Anticorps IgG1 (titre)^{c} | 0,7x10⁵ | 10x10⁵ | 8x10⁵ | 30x10⁵ |

| | | | | |
|---|---|---|---|---|
| ^{a} Moyenne du nombre de cellules sécrétant de l'IFN-γ / 10⁶ cellules totales, en réponse à un stimulus spécifique (peptide AMQMLKETI) de 20h ^{b} Nombre de souris ayant une activité CTL spécifique sur le nombre de souris total du bras ^{c} Moyenne géométrique des titres IgG1 anti-p24 des souris du bras | | | | |

Les résultats dans le tableau mettent en évidence que :
- si on compare les titres en anticorps reportés dans le tableau 1 et ceux reportés ici dans le tableau 2, augmenter la dose de 10 µg à 40 µg permet de donner des résultats comparables, et
- une réponse à plus long terme après la dernière injection, traduite par un sacrifice plus tardif des souris, permet de mettre en évidence une réponse CTL chez toutes les souris du groupe p24/PLA, ainsi qu'une réponse en ELISPOT et en titres d'anticorps améliorés.

### Exemple 4 : Immunisation de souris avec les microparticules de l'invention PLA/Tat

On a répété le mode opératoire indiqué dans l'exemple 3, à ceci près qu'on a utilisé comme immunogène les microparticules de PLA/Tat telles que préparées dans l'exemple 1, point 3 ci-dessus, la protéine Tat seule et la combinaison protéine Tat/adjuvant de Freund (Sigma) préparée sous forme d'émulsion eau-dans-huile,que les dose d'injection étaient chacune de 20 µg, que pour la réponse humorale, on a utilisé en tant que partenaire de capture la protéine Tat et en tant que partenaire de détection les anticorps polyclonaux de souris tels qu'indiqués dans l'exemple 3, point 4 ci-dessus, et que pour la réponse humorale, on a effectué uniquement un test ELISPOT en utilisant comme stimulus soit les 6 peptides tels qu'indiqués ci-après, pendant 20h pour analyser les réponses de type CD8, soit la protéine Tat pendant 42h pour analyser les réponses de type CD4.

**Peptides utilisés dans le test ELISPOT (Sygma Genosys)**

| | |
|---|---|
| CFHCQVCFTKKGLGI (SEQ ID N°4) | SYGRKKRRQRRRSPQ (SEQ ID N°7) |
| VCFTKKGLGISYGRK (SEQ ID N°5) | KRRQRRRSPQDSETH (SEQ ID N°8) |
| KGLGISYGRKKRRQR (SEQ ID N°6) | RRSPQDSETHQVSLS (SEQ ID N°9) |

Les résultats sont indiqués dans le tableau 3 ci-dessous.

**Tableau 3**

| | Tat seule | Tat/Freund | Tat/PLA |
|---|---|---|---|
| ELISPOT IFN-γ CD8^{a} | 3 | 3 | 24 |
| ELISPOT IFN-γ CD4^{b} | 4 et 6 | 4et8 | 28 et 50 |
| Anticorps IgG (titre)^{c} | 0,1x10⁵ | 0,2x10⁵ | 1,7x10⁵ |
| Anticorps IgG2a (fréquence)^{d} | 2/4 | 1/4 | 3/4 |
| Anticorps IgG2a (titre)^{e} | 730 | 560 | 3900 |

| | | | |
|---|---|---|---|
| ^{a} Moyenne du nombre de cellules sécrétant de l'IFN-γ / 10⁶ cellules totales, en réponse à un stimulus spécifique (pools de peptide) de 20h ^{b} Moyenne du nombre de cellules sécrétant de l'IFN-γ / 10⁶ cellules totales, en réponse à un stimulus spécifique (protéine Tat) de 42h ^{c} Moyenne géométrique des titres IgG totales anti-Tat des souris du bras ^{d} Nombre de souris ayant une réponse IgG2a spécifique sur le nombre de souris total du bras ^{e} Moyenne géométrique des titres IgG2a anti-Tat des souris qui ont répondu | | | |

Les résultats dans le tableau 3 ci-dessus mettent en évidence que :
- l'injection des microparticules de l'invention permet d'induire des cellules sécrétant de l'IFN-γ alors que l'injection de la protéine Tat seule ou de la composition Tat/Freund et
- la liaison de la protéine Tat sur les PLA permet d'améliorer les titres en anticorps (IgG totales) d'environ 1 log par rapport aux titres obtenus avec la protéine Tat seule ou adjuvantée en Freund qui sont de l'ordre de 10⁴. L'utilisation des microparticules Tat/PLA permet d'améliorer à la fois la fréquence et le titre des IgG2a anti-Tat.

### Exemple 5 : Immunisation de souris avec les microparticules de l'invention PLA/NS3 hélicase

### 1. Modèle animal

Les expériences d'immunisation ont été réalisées chez 14 souris C57BL/6 trangéniques pour la molécule HLA-A2 (Pascolo S., et al. (1997), J. Exp Med., 185(12), 2043-2051).

### 2. Immunogènes administrés

Dans cette expérience, on a utilisé de l'ADN nu correspondant à la séquence en acides nucléiques NS3NS4 (SEQ ID N°10) à titre de témoin positif, le peptide NS3 hélicase seul, les particules de PLA seules, telles que préparées dans l'exemple 1, points 1 et 4 ci-dessus, la composition NS3 hélicase/adjuvant de Freund (Sigma) préparée sous forme d'émulsion eau-dans-huile , ainsi que les microparticules de l'invention PLA/NS3 hélicase telles que préparées dans l'exemple 1, point 4 ci-dessus.

### 3. Immunisations

Les souris ont reçu 3 doses successives des immunogènes décrits dans le point 2 ci-dessus, à 0, 2 et 4 semaines, à raison de 50 µg chacune dans le cas des protéines ou de 100 µg chacune dans le cas de l'ADN nu. Toutes les injections ont été réalisées par voie sous-cutanée, à l'exception de l'ADN nu qui a été administré par voie intramusculaire.

Les animaux ont été sacrifiés environ 70 jours (J70) après la première injection et le sang et la rate ont été prélevés pour les analyses immunologiques

### 4. Analyse immunologiques

On a recherché la réponse cellulaire CTL comme suit : après sacrifice des souris, les rates ont été prélevées de façon stérile pour préparer une suspension cellulaire. La suspension cellulaire a été mise en culture en présence du peptide KLV (KLVALGVNAV, SEQ ID N°11), qui correspond à un épitope CTL contenu dans la protéine NS3, et d'IL-2. Cinq jours plus tard, la population effectrice a été restimulée par des cellules naïves irradiées, chargées avec le peptide. La population cytotoxique effectrice a été récoltée au bout du 7^{ème} jour et l'activité CTL a été mesurée en utilisant comme cibles les cellules P815 marquées au ⁵¹Cr.

### 5. Résultats

Les résultats sont indiqués sur la figure 1 représentant des graphes donnant le pourcentage de lyse spécifique en fonction du ratio effecteurs/cibles et où la figure 1A donne la réponse cellulaire induite après injection de la séquence d'ADN correspondant à la polyprotéine NS3NS4 du VHC à titre de témoin, la figure 1B donne la réponse cellulaire induite après injection de la combinaison NS3 hélicase/adjuvant de Freund sans microparticule, la figure 1C donne la réponse cellulaire induite après injection du peptide NS3 hélicase sans microparticule, la figure 1D donne la réponse cellulaire induite après injection des microparticules de PLA sans substance protéique, et la figure 1E donne la réponse cellulaire induite après injection des microparticules de l'invention PLA/NS3 hélicase.

Ces graphes montrent une réponse CTL spécifique du peptide NS3 hélicase, mise en évidence lorsqu'on injecte les microparticules PLA/NS3 de l'invention.

### Exemple 6 : Immunisation de lapins par les microparticules de l'invention PLA/p24

### 1. Modèle animal

Les expériences d'immunisation ont été réalisées chez des lapins de la souche New Zealand White pesant environ 2,5 kg au moment de la première immunisation.

### 2. Immunogènes administrés

Dans cette expérience, on a utilisé les microparticules de l'invention PLA/p24 préparées comme indiqué dans l'exemple 1, point 2 ci-dessus, ainsi que la composition p24-adjuvant de Freund (Sigma) préparée sous forme d'émulsion eau-dans-huile et dont il est connu qu'elle présente un bon pouvoir immunogène (témoin positif).

### 3. Immunisations

Les lapins ont reçu 5 doses successives de 200 µg des immunogènes décrits dans le point 2 ci-dessus à 0, 1, 2, 3 et 4 mois. Toutes les injections ont été réalisées par voie sous-cutanée ou par voie intradermique.

### 4. Suivi de l'apparition de la réponse humorale anti-p24

Afin de suivre l'apparition des anticorps anti-p24, on a effectué régulièrement sur les animaux des prélèvements de sang. La présence des anticorps anti-p24 a alors été testée en utilisant le test ELISA similaire à celui décrit dans l'exemple 3, point 4, à ceci près que le conjugué de révélation a été remplacé par un anticorps de chèvre anti-IgG de lapin AffiniPure conjugué à la peroxydase de raifort (H+L, Jackson Immunoresearch, Cat no. 111-035-003).

### 5. Résultats

Les résultats sont indiqués sur la figure 2 qui donne les titres en IgG anti-p24 dans les sérums preimmuns et les sérums immuns des 6 lapins (L1 à L6) ayant reçu une injection soit par voie intradermique (ID), soit par voie sous-cutanée (SC). Le titre correspond à l'inverse de la dilution pour laquelle on obtient une DO environ égale à 0,1. Le sérum pré-immun a été prélevé à J0, avant l'injection des immunogènes et le sérum immun a été prélevé à J0 plus 4 mois.

Les résultats obtenus montrent que l'immunisation avec les particules PLA/p24 de l'invention donne de bons titres chez tous les animaux, que l'antigène ait été administré par voie sous-cutanée ou intradermique. Cependant dans le modèle choisi, la voie intradermique semble un peu supérieure à la voie sous-cutanée. On peut noter que les titres obtenus par l'immunisation de Freund/p24 sont sensiblement comparables à celles obtenues par une immunisation PLA/p24 (2 x 10⁷ *vs* 5 x 10⁶). Les microparticules PLA/p24 peuvent donc être utilisées pour induire un sérum polyclonal chez le lapin.

### Exemple 7 : Immunisation de macaques par les microparticules de l'invention PLA/p24

### 1. Modèle animal

Les expériences d'immunisation ont été réalisées chez des macaques cynomolgus hébergés au CEA.

### 2. Immunogènes administrés

Dans cette expérience, on a utilisé les microparticules de l'invention PLA/p24 préparées comme indiqué dans l'exemple 1, point 2 ci-dessus.

### 3. Immunisations

Deux macaques cynomolgus ont été immunisés à l'aide d'une injection de PLA/p24 (500 µg par animal en IM), suivie d'un rappel identique 6 semaines plus tard. Une troisième injection est réalisée dans les mêmes conditions 6 mois plus tard.

### 4. Suivi de l'apparition de la réponse humorale anti-p24

Afin de suivre l'apparition des anticorps anti-p24, on a effectué sur les macaques des prélèvements de sang les semaines 0 (le jour de la primo vaccination), 2, 4, 6 (le jour de l'injection de rappel), 8, 10, 12. La présence des anticorps anti-p24 a alors été testée en utilisant le test ELISA similaire à celui décrit dans l'exemple 3, point 4 ci-dessus, à ceci près qu'on a remplacé le conjugué de révélation par un anticorps de souris anti-fragment Fc gamma d'IgG humain AffiniPure conjugué à la peroxydase de raifort (H+L, Jackson Immunoresearch, Cat no. 209-035-098). Le même format d'ELISA a aussi été utilisé pour analyser les sous-classes d'IgG présentes, en utilisant des anticorps conjugués à la peroxydase de raifort anti-IgG1 humain (Cat no. 05-3320, Zymed), anti-IgG2 humain (Cat no. 05-0520, Zymed), anti-IgG3 humain (Cat no. 05-3620, Zymed), anti-IgG4 humain (Cat no. 05-3820, Zymed).

### 5. Suivi de l'apparition de la réponse cellulaire anti-p24 par ELISPOT

Ce mode opératoire permet de déterminer le nombre de cellules sécrétant de interféron gamma (IFN-gamma) en réponse à une stimulation antigénique à la concentration finale de 5µg/ml pendant 48h. Ce mode opératoire a été utilisé avec succès avec des cellules mononucléées du sang périphérique (CMSP) fraîchement isolées, des CMSP préalablement cryoconservées, des lignées de lymphocytes T issues de CMSP stimulées *in vitro* et de CMSP préalablement déplétées en cellules CD4⁺ (utilisation d'un anticorps anti-CD4) ou CD8⁺ (utilisation d'un anticorps anti-CD8). Le tri cellulaire CD4⁺ ou CD8⁺ a été effectué en utilisant les réactifs MACS, CD4 microbeads (Cat no. 130-091-102) et CD8 microbeads kit (Cat no. 130-091-112) de Miltenyi Biotec, selon les instructions du fabricant

Le couple PMA-ionomycine (PMA pour Phorbol Myristate Acétate), qui mime l'effet d'une activation antigénique des lymphocytes T, a été utilisé en tant que témoin positif.

Les plaques d'ELISPOT 96 puits avec des membranes de PVDF (Multiscreen, Millipore) ont été coatées par l'anticorps monoclonal anti-IFN gamma de macaque, clone GZ-4 (Mabtech, ref: 3420M-3) à 1µg/ml dans du PBS stérile, sur la nuit à +4°C. Les plaques ont ensuite été lavées et saturées. Parallèlement, les CMSP ont été isolées à partir des prélèvements de sang sur gradient de Ficoll, selon les techniques habituelles. On a déposé 10⁵ cellules dans 100 µl de milieu de culture/puits et la source d'antigène dans 100 µl de milieu de culture/puits. Selon les expériences, la source d'antigène est soit la protéine p24, soit un pool de peptides gag tel que définis ci-après et dont on a vétifié au préalable qu'ils permettaient d'obtenir une réponse positive en ELISPOT. Pour réaliser des témoins positifs de stimulation, on a déposé 4 × 10³ cellules dans 200 µl de milieu de culture/puits contenant 50 ng/ml de PMA et 500 ng/ml de ionomycine. Les plaques ont alors été incubées 24 h à 37°C en atmosphère humide à 5 % CO₂, puis lavées au PBS. Les cellules restantes ont alors été lysées par un traitement à l'eau glacée pendant 10 minutes, puis les plaques ont encore été lavées. On a ensuite ajouté l'anticorps de révélation, le monoclonal biotinylé dirigé contre l'IFN-gamma humain, clone 7-B6-1 (Mabtech, ref : 3420-6) à 0,1µg/puits (incubation 2h à 37°C ou 1 nuit à 4°C). Les spots ont été révélés par l'ajout d'extravidine-phosphatase alcaline et du substrat 5 bromo-4-chloro-3-indolyl phosphate/nitro blue tetrazolium (BCIP/NBT). Les séquences des peptides gag utilisés sont les suivantes :
EQIGWMTNNPPIPVG (SEQ ID N°12)
WMTNNPPIPVGEIYK (SEQ ID N°13)
NPPIPVGEIYKRWII (SEQ ID N°14)
PVGEIYKRWIILGLN (SEQ ID N°15)
IYKRWIILGLNKIVR (SEQ ID N°16)
WIILGLNKIVRMYSP (SEQ ID N°17)
GLNKIVRMYSPTSIL (SEQ ID N°18)
IVRMYSPTSILDIRQ (SEQ ID N°19)
YSPTSILDIRQGPKE (SEQ ID N°20)
SILDIRQGPKEPFRD (SEQ ID N°21)
IRQGPKEPFRDYVDR (SEQ ID N°22)
PKEPFRDYVDRFYKT (SEQ ID N°23)
FRDYVDRFYKTLRAE (SEQ ID N°24)
VDRFYKTLRAEQASQ (SEQ ID N°25)
YKTLRAEQASQEVKN (SEQ ID N°26)
RAEQASQEVKNWMTE (SEQ ID N°27)
ASQEVKNWMTETLLV (SEQ ID N°28)
VKNWMTETLLVQNAN (SEQ ID N°29)
MTETLLVQNANPDCK (SEQ ID N°30)
LLVQNANPDCKTILK (SEQ ID N°31)
NANPDCKTILKALGP (SEQ ID N°32)

### 6. Résultats

### 6.1 Mise en évidence des réponses ELISPOT

Les résultats des tests ELISPOT IFN-gamma des deux macaques (M1 et M2) ayant reçu la préparation de PLA/p24 et dont les CMSP ont été stimulées sont reproduits sur la figure 3 qui représente des histogrammes (moyenne de 4 réplicats +/- écarts type) donnant le nombre de spots par millions de cellules obtenu en fonction des jours post vaccination après stimulation sans antigène (milieu, témoin négatif, histogramme plein), après stimulation avec la p24 (histogramme rayé) ou après stimulation par le couple PMA Ionomycine. Les flèches sous chaque graque représente le moment des injections (J0 et J + 6 semaines).

Ces résultats mettent en évidence que les microparticules de l'invention PLA/p24 permettent d'induire une réponse ELISPOT IFN-gamma chez les deux animaux testés. Le premier singe (M1) ne développe une réponse spécifique qu'après la deuxième injection alors que le second animal (M2) a déjà développé une réponse au premier prélèvement testé, après la première injection. Dans les deux cas, l'effet de l'injection de rappel est très significatif (effet boost) puisque les réponses Elispot obtenues sont élevées. De plus, les réponses obtenues sont relativement durables dans le temps, compte tenu du fait qu'il s'agit d'un immunogène non réplicatif ; les réponses restent significatives environ 40 jours après la deuxième injection.

### 6.2 Nature des réponses ELISPOT

La nature des réponses ELISPOT permet de mettre en évidence quels types de cellules immunitaires effectrices, CD4+ ou CD8+, sont responsables de la sécrétion d'IFN-gamma mesurée

Les résultats sont donnés sur la figure 4 qui représente des histogrammes donnant le nombre de spots obtenus en ELISPOT après stimulation soit avec la protéine p24 (histogrammes pleins), soit avec les peptides (histogrammes rayés), dans la fraction de CMSP totales, la fraction de CMSP totales en présence de l'anticorps anti-CD4, la fraction de CMSP déplétée en CD4⁺ (CMSP CD4-) et la traction enrichie en CD4⁺ correspondante (CMSP CD4+), ainsi que la fraction de CMSP déplétée en CD8⁺ (CMSP CD8-) et la fraction enrichie en CD8⁺ correspondante (CMSP CD8+). Il est à noter que la quantité de cellules obtenues chez le singe M1 n'était pas suffisante pour effecteur les expériences de déplétion par l'anticorps anti-CD8.

Les résultats obtenus sur la figure 4 montrent que la sécrétion de la cytokine IFN-gamma est effectuée à la fois par la fraction CD4- et la fraction CD8- des cellules mononuclées du sang périphérique. De plus, il n'y pas de réponse Elispot en présence d'un anticorps anti-CD4 ou d'un anticorps anti-CD8 (fractions de tri positives) indiquant que chacun de ces anticorps interfère avec la réponse immune. Cette expérience permet donc de conclure que la sécrétion d'IFN-gamma observée est médiée à la fois par des cellules effectrices CD4+ et des cellules effectrices CD8+. De plus, cette observation est valide quelle que soit la nature de la stimulation antigénique utilisée, protéine p24 ou pool de peptides gag. Il est important de souligner que c'est la première fois qu'une formulation à base de microparticules permet d'induire chez des primates une réponse CD8+ spécifique de l'antigène d'intérêt.

### 6.3 Analyse de la réponse humorale

En même temps que l'analyse des réponses cellulaires sur les CMSP, nous avons aussi effectué l'analyse de la réponse humorale sur les sérums séquentiels obtenus chez les singes. Parmi les deux animaux uniquement le singe M2 a développé une réponse anticorps qui a atteint un titre en IgG anti-p24 de 10⁵ environ suivant la deuxième injection. Le singe M1 n'a pas développé de réponse anticorps significatif après deux injections de PLA/p24, le titre restant inférieur à 10³. Ce résultat n'est pas très étonnant car lors de l'analyse des réponses cellulaires nous avons pu mettre en évidence que le singe M1 était un mauvais répondeur, nécessitant une injection supplémentaire par rapport au singe M2 pour développer une réponse immune.

Les résultats du test ELISA avec le singe M2 sont donnés sur la figure 5 qui représente des histogrammes donnant le titre en IgG anti-p24 chez le singe M2 en fonction des jours de prélèvement des sérums séquentiels. Ces résultats montrent d'une part qu'il est possible d'induire des réponses anticorps spécifiques chez les macaques avec les microparticules de l'invention PLA/p24 et d'autre part qu'il faut très probablement ajouter une ou deux injections supplémentaires au protocole que nous avons utilisé pour être certain d'induire une réponse même chez les répondeurs les moins bons.

### 6.4 Conclusion

L'ensemble de ces expériences réalisées dans le modèle du macaque cynomolgus a permis de monter que les microparticules de l'invention permettent d'induire de bonnes réponses immunes chez un primate non-humain, avec des réponses cellulaires CD4+, CD8+ et des réponses anticorps.

### Exemple 8: Immunisation de souris par des cellules dendritiques sensibilisées par les microparticules de l'invention PLA/p24

### 1. Modèle animal

Les expériences d'immunisation ont été réalisées chez des souris CBA/J (H-2^{k}) femelles âgées de 6 à 8 semaines au moment de la première immunisation.

### 2. Immunogènes administrés

Dans cette expérience, on a utilisé la lignée de cellules spléniques murines SRDC (H-2^{k}) afin de véhiculer les différents immunogènes à tester. Cette lignée de cellules dendritiques a été sensibilisée pendant 12h avec l'un des immunogènes suivants : microparticules de l'invention PLA/p24 préparées comme indiqué dans l'exemple 1, point 2 (à 10 µg/ml de p24/PLA en équivalent p24), microparticules PLA-OVA, préparées de la même façon qui vont servir de témoin négatif, ou protéine p24.

### 3. Immunisations

Les souris ont été divisées en 4 lots de 6 animaux. Chaque souris a reçu 5x 10⁵ cellules SRDC sensibilisées à 0, 2 et 4 semaines par voie sous-cutanée. Les 4 lots de souris ont reçu soit des cellules non sensibilisées (témoin négatif), soit sensibilisées par les microparticles PLA/p24 ou PLA/OVA (témoin négatif microparticules) ou la protéine p24.

### 4. Suivi de l'apparition de la réponse humorale anti-p24

Afin de suivre l'apparition des anticorps anti-p24, on a effectué sur les souris des prélèvements de sang tous les 10 jours après immunisation. La présence des anticorps anti-p24 a alors été testée en utilisant le test ELISA similaire à celui décrit dans les exemples précédents.

### 5. Résultats

L'analyse de la réponse humorale est présentée sur la figure 6 représente des histogrammes qui donnent la valeur de DO obtenue en ELISA dans le sérum préimmun, dans le sérum après la 1^{ère} immunisation, dans le sérum après la deuxième immunisation et dans le sérum après la 3^{ème} immunisation, des souris sensibilisées soit par le témoin négatif (Tm1 et Tm2), soit par la protéine p24 (p24-1 et p24-2), soit par le témoin négatif microparticules (NanoOva1 et NanoOva2) et soit par les microparticules de l'invention (Nanop24-1 et Nanop24-2).

Les résultats montrent que l'injection des cellules SRDC n'induit d'anticorps que lorsque les SRDC sont sensibilisées par les microparticules PLA-p24. La sensibilisation par la protéine p24 soluble ne permet pas d'obtenir de réponse immune.

### 6. Etude de la réponse cellulaire

Cette étude a été réalisée à la fois à partir des lymphocytes spléniques et muqueux isolés des souris. L'analyse des sous-populations lymphocytaires T stimulées a été faite par des tests de lymphoprolifération et de sécrétion de cytokines de façon à évaluer leur capacité à répondre à l'antigène, 2 semaines après la dernière immunisation.

Pour les tests de lymphoprolifération, les lymphocytes T isolés de la rate et des ganglions muqueux ont été mis en culture pendant 5 jours dans du milieu de culture complet en présence de différentes concentrations de l'antigène d'intérêt puis radiomarquées avec de la thymidine ³H pendant 18h. Le niveau d'incorporation de la thymidine correspond au degré de la réponse lymphoproliférative.

Pour les tests de sécrétion de cytokines, les lymphocytes T isolés de la rate et des ganglions muqueux ont été mis en culture pendant 3 jours dans du milieu de culture complet en présence de différentes concentrations de l'antigène d'intérêt puis les cytokines sécrétées dans le surnageant de culture ont été dosées par des kits ELISA du commerce.

### 7. Résultats

Le tableau 4 ci-après donnent un récapitulatif des résultats obtenus.

**Tableau 4**

| Groupes de souris | Nombre de souris ayant développé une réponse anticorps (IgG) après la 3^{e} injection | Lymphoprolifération Index de stimulation Rate-Ganglion | Sécrétion d'IFN-gamma |
|---|---|---|---|
| Témoin | 0/2 | 1 - 1 | non |
| SRDC-p24 | 0/2 | 1 - 1 | non |
| SRDC-PLA-Ova | 0/2 | 1,4 - 1,4 | non |
| SRDC-PLA-p24 | 2/2 | 2,8 - 2,2 | + rate, ++ ganglion |

Comme précédemment, les résultats montrent que le groupe SRDC-PLA-p24 est le seul groupe pour lequel il est possible de mettre en évidence une réponse immune spécifique. L'index de stimulation est de 2,8 pour les cellules isolées de la rate et de 2,2 pour celles isolées des ganglions mésentériques. Pour les autres groupes il n'y a pas d'augmentation de l'index de stimulation ou elle n'est pas significative. De la même manière le groupe SRDC-PLA-p24 est le seul groupe qui permet d'induire une sécrétion d'IFN-gamma, cytokine de type Th1. Cette sécrétion en présence de l'antigène peut être mise en évidence à la fois à partir des cellules de rate et de celles des ganglions mésentériques.

### 8. Conclusion

L'ensemble de ces expériences nous a permis de monter qu'il est aussi possible d'utiliser les microparticules PLA-p24 afin de sensibiliser les cellules dendritiques par un antigène d'intérêt, en l'occurrence la p24. Alors que l'administration des SRDC permet d'induire à la fois des réponses cellulaires et humorales spécifiques, lorsqu'elles sont chargées par les PLA-p24, les SRDC sensibilisées par la p24 soluble ne permettent pas d'induire de réponses anti-p24. Ainsi, les microparticules PLA peuvent également être utilisées avec succès dans les applications d'immunothérapie basées sur le transfert de cellules dendritiques sensibilisées *in vitro.* Lorsque l'antigène intérêt ne peut pas être chargé sous sa forme soluble dans les cellules dendritiques, les microparticules de PLA portant l'antigène peuvent être utilisées pour faciliter sa capture par les cellules dendritiques. L'utilisation des microparticules de l'invention permet d'améliorer considérablement les réponses immunes spécifiques obtenues.

### Exemple 9: Immunisation de souris avec les microparticules de l'invention PLA/NS3 hélicase : Réponse cellulaire précoce et localisée

### 1. Modèle animal

Les expériences d'immunisation ont été réalisées chez les souris BALB/c (H-2^{d}) femelles âgées de 6 à 8 semaines au moment de la première immunisation.

### 2. Immunogènes administrés

Dans cette expérience, on a utilisé 5 souris par groupe d'immunisation : la protéine NS3 hélicase génotype 1b (NS3h) seule, les microparticules de l'invention PLA/NS3h préparées par dialyse (PLADYS) comme indiqué dans l'exemple 1, point 4 ci-dessus, les microparticules de l'invention PLA/NS3h préparées par déplacement de solvant (PLADDS) comme indiqué dans l'exemple 2, point 2 ci-dessus, ainsi que la composition NS3h-Alum (Pierce) préparée sous forme d'émulsion et connue comme adjuvant dans les vaccins commerciaux (témoin positif).

### 3. Immunisations

Les souris ont reçu 1 dose (100 µg) des immunogènes décrits dans le point 2 ci-dessus par voie sous cutanée dans le coussinet plantaire.

Les animaux ont été sacrifiés 10 jours après la première injection et les ganglions poplités ont été prélevés pour l'analyse immunologique.

### 4. Analyses immunologiques

On a recherché la réponse cellulaire dose - réponse spécifique à la protéine NS3h précoce et localisée comme suit :
*Prolifération des cellules des ganglions poplités après stimulation avec différentes concentrations de la protéine NS3h -* les cellules des ganglions poplités ont été stimulées en présence de 0; 0,1 ; 0,3 ; 1 µM de la protéine NS3h pendant 3 jours. Les cellules ont été pulsées pendant 18h par la thymidine tritiée, qui s'incorpore dans l'ADN des cellules qui sont en train de proliférer. A la suite du pulse, les cellules ont été récoltées sur une membrane qui retient l'ADN et permet d'éliminer par lavage la thymidine marquée non incorporée. Plus les cellules prolifèrent en réponse au stimulus spécifique, plus l'ADN est marqué ; en d'autres termes, plus la réponse cellulaire contre l'immunogène NS3h est importante.

### 5. Résultats

Les résultats de la prolifération des cellules des ganglions poplités après stimulation avec la protéine NS3h sont indiqués sur la figure 7 représentant les graphes donnant l'indice de prolifération relative (IPR) spécifique à la protéine NS3h, correspondant au ratio de cpm (coup par min) obtenu pour chaque concentration de NS3h (0 ; 0,1 ; 0,3 et 1 µM) par rapport à la concentration zéro de la protéine NS3h, en fonction de la quantité de NS3h utilisée pour la restimulation pour le test de prolifération cellulaire, chez des souris ayant reçu du NS3h-PBS (PBS), des microparticules de l'invention préparée par dialyse (DYS), des microparticules de l'invention préparée par déplacement de solvant (DDS) et la composition NS3h-Alum.

Ce graphe montre une réponse cellulaire spécifique de la protéine NS3h lorsqu'on injecte les microparticules PLA/NS3h préparées par dispersion de solvant (DDS) et PLA/NS3h préparées par dialyse (DYS). Les réponses cellulaires spécifiques des souris injectées avec les PLADDS/NS3h de l'invention est plus forte que celles obtenues avec les PLADYS/NS3h de l'invention, le témoin positif (Alum/NS3h) et le contrôle PBS/NS3h pour toutes les concentrations d'antigènes NS3h testés (PLADDS /NS3h > PLADYS/NS3h > Alum/NS3h = PBS/NS3h).

Les résultats montrent qu'on obtient des réponses prolifératives cellulaires améliorées avec les microparticules de l'invention PLADDS/NS3h et PLADYS/NS3h, par rapport à la protéine NS3h seule ou adjuvantée par du Alum.

### Exemple 10: Immunisation de souris avec les microparticules de l'invention PLA/NS3 hélicase : Réponse cellulaire localisée et systémique et inhibition de la réponse cellulaire systémique avec des anticorps anti-CD4⁺

### 1. Modèle animal

Les expériences d'immunisation ont été réalisées chez les souris BALB/c (H-2^{d}) femelles âgées de 6 à 8 semaines au moment de la première immunisation.

### 2. Immunogènes administrés

Dans cette expérience, on a utilisé 5 souris par groupe d'immunisation : la protéine NS3 hélicase génotype 1b (NS3h) seule, les microparticles de l'invention PLA/NS3h préparées par dialyse (PLADYS) comme indiqué dans l'exemple 1, point 4 ci-dessus, les microparticules de l'invention PLA/NS3h préparées par déplacement de solvant (PLADDS) comme indiqué dans l'exemple 2, point 2 ci-dessus, ainsi que la composition NS3h-Alum (Pierce) préparée sous forme d'émulsion et connue comme adjuvant dans les vaccins commerciaux (témoin positif).

### 3. Immunisations

Les souris ont reçu 2 doses (100 µg) des immunogènes décrits dans le point 2 ci-dessus, la première dose ayant été réalisée par voie sous cutanée dans le coussinet plantaire à jour 0 et la deuxième par voie sous-cutanée à la base de la queue à jour 7.

Les animaux ont été sacrifiés 7 jours après la deuxième injection et les ganglions poplités et la rate ont été prélevés pour l'analyse immunologique.

### 4. Analyses immunologiques

On a recherché la réponse cellulaire dose - réponse spécifique à la protéine NS3h localisée dans les ganglions poplités et systémiques dans la rate comme suit :
*Prolifération des cellules des ganglions poplités après stimulation avec différentes concentrations de la protéine NS3h -* les cellules des ganglions poplités et de la rate ont été stimulées en présence de 0; 0,1 ; 0,3 ; 1 µM de la protéine NS3h pendant 3 jours. Après 3 jours de culture, on a prélevé 50 µl de surnageant. Les cellules ont été pulsées pendant 18h par la thymidine tritiée, qui s'incorpore dans l'ADN des cellules qui sont en train de proliférer. À la suite du pulse, les cellules ont été récoltées sur une membrane qui retient l'ADN et qui permet d'éliminer par lavage la thymidine marquée non incorporée. Plus les cellules prolifèrent en réponse au stimulus spécifique, plus l'ADN est marqué ; en d'autres termes, plus la réponse cellulaire contre l'immunogène NS3h est importante.

On a recherché à inhiber la réponse cellulaire avec des anticorps anti-CD4⁺ (GK1.5 ; American Type Culture Collection (ATCC)) dans les cellules provenant de la rate comme suit :
(i) *Inhibition avec l'anticorps anti-CD4*⁺ *de la prolifération des cellules de la rate après stimulation avec 1*µ*M de la protéine NS3h -* les cellules de la rate ont été stimulées en présence de 1 µM de la protéine NS3h et incubées avec 10 µg d'anticorps anti-CD4⁺ pendant 3 jours. Les cellules ont été pulsées pendant 18h par la thymidine tritiée, qui s'incorpore dans l'ADN de cellules qui sont en train de proliférer. À la suite du pulse, les cellules ont été récoltées sur une membrane qui retient l'ADN et qui permet d'éliminer par lavage la thymidine marquée non incorporée. Plus les cellules prolifèrent en réponse au stimulus spécifique, plus l'ADN est marqué ; en d'autres termes, plus la réponse cellulaire contre l'immunogène NS3h est importante.
(ii) *Dosage de l'interféron* γ *-* L'interféron γ a été dosé à l'aide du kit BD™ Cytometric Bead Array , Mouse Th1/Th2 Cytokine CBA (BD Biosciences, Cat. N°. 551287). Cinq populations de billes avec des intensités distinctes de fluorescence ont été cotées par des anticorps de capture spécifiques pour IL-2, IL-4, IL-5, IFN-gamma, et protéines de TNF-alpha. Les cinq populations de billes ont été mélangées ensemble pour former le CBA, qui est résolu dans le canal FL3 d'un cytomètre à écoulement tel que le BD FACScanTM Coule Cytometer. Les billes de capture de cytokine ont été mélangées à des anticorps de détection conjugués à la phycoérythrine, et puis incubées selon les recommendations du fabricant. L'acquisition des données d'échantillon en utilisant le cytomètre d'écoulement pour produire les résultats a été effectuée en utilisant le logiciel d'analyse de BD CBA.

### 5. Résultats

### 5.1 Réponse cellulaire localisée et systémique

Les résultats de la prolifération des cellules de la rate après stimulation avec la protéine NS3h sont indiqués sur la figure 8 représentant les graphes donnant l'indice de prolifération relative (IPR) spécifique à la protéine NS3h en fonction de la quantité de NS3h utilisée (en µM) chez des souris ayant reçu du NS3h-PBS (PBS), des microparticules de l'invention préparée par dialyse (DYS), des microparticules de l'invention préparée par déplacement de solvant (DDS) et la composition NS3h-Alum (Alum) dans les cellules des ganglions poplités pour la réponse cellulaire localisée (figure 8A) et dans les cellules de la rate pour la réponse cellulaire systémique (figure 8B).

Ces résultats mettent en évidence une réponse cellulaire systémique (cellules de la rate- figure 8B) et spécifique de la protéine NS3h à 1µM avec les microparticules PLA/NS3h de l'invention préparées par dispersion de solvant (DDS) et PLA/NS3h préparées par dialyse (DYS). Les réponses cellulaires spécifiques des souris ayant reçu les PLADDS/NS3h et PLADYS/NS3h de l'invention de l'invention sont plus fortes que celles obtenues avec le témoin positif (Alum/NS3h) et le contrôle PBS/NS3h (PBS) (PLADDS/NS3h et PLADYS/NS3H >>> Alum/NS3h = PBS/NS3h).

### 5.2 Inhibition de la réponse cellulaire systémique avec l'anticorps anti-CD4

Les résultats sont indiqués sur la figure 9 qui représente des histogrammes donnant l'indice de prolifération cellulaire (IPR) en fonction des immunogènes utilisés chez des souris, à savoir du NS3h-PBS (PBS), des microparticules de l'invention préparées par dialyse (DYS), des microparticules de l'invention préparées par déplacement de solvant (DDS) et la composition NS3h-Alum (Alum), dans les cellules de la rate sans stimulation (0), après stimulation avec de la protéine NS3h (1) ou après stimulation avec de la protéine et de l'anticorps anti-CD4+ (1+aCD4).

Les résultats montrent que la réponse cellulaire systémique est inhibée d'au moins un facteur 5 en présence de l'anticorps anti-CD4+, suggérant que la réponse spécifique à la protéine NS3h est du type Th2.

### 5.3 Résultats complémentaires

Au troisième jour de la prolifération après la stimulation de la protéine NS3h, on a observé une sécrétion de l'Interféron gamma (IFN-gamma) dans toutes les conditions utilisées qui est diminuée avec l'anticorps anti-CD4⁺.

Les résultats du dosage de l'IFN-gamma au troisième jour après la stimulation de la protéine NS3h sont donnés dans le tableau 5 ci-dessous :

**Tableau 5**

| | Type de stimulation | Dosage de l'interféron gamma pg/ml |
|---|---|---|
| PBS/NS3h | 1µM de NS3h | 316 |
| | 1µM de NS3h + anti-CD4+ | 60 |
| PLADDS/NS3h | 1µM de NS3h | 556 |
| | 1µM de NS3h + anti-CD4+ | 196 |
| PLADYS/NS3h | 1µM de NS3h | 606 |
| | 1µM de NS3h + anti-CD4+ | 249 |
| Alum/NS3h | 1µM de NS3h | 583 |
| | 1µM de NS3h + anti-CD4+ | 144 |

### Exemple 11 : Immunisation de souris avec les microparticules de l'invention PLA/NS3 hélicase : Réponse humorale

### 1. Modèle animal

Les expériences d'immunisation ont été réalisées chez les souris BALB/c (H-2^{d}) femelles âgées de 6 à 8 semaines au moment de la première immunisation.

### 2. Immunogènes administrés

Dans cette expérience, on a utilisé 5 souris par groupe d'immunisation : la protéine NS3 hélicase génotype 1b (NS3h) seule, les microparticules de l'invention PLA/NS3h préparées par dialyse (PLADYS) comme indiqué dans l'exemple 1, point 4 ci-dessus, les microparticules de l'invention PLA/NS3h préparées par déplacement de solvant (PLADDS) comme indiqué dans l'exemple 2, point 2 ci-dessus, ainsi que la composition NS3h-Alum (Pierce) préparée sous forme d'émulsion et connue comme adjuvant dans les vaccins commerciaux (témoin positif).

### 3. Immunisations

Les souris ont reçu 3 doses (50 µg) des immunogènes décrits dans le point 2 ci-dessus par voie sous cutanée à la base de la queue à 0, 2 et 4 semaines. Les sera ont été prélevés à jour 13, jour 27 et jour 45 pour les analyses de la réponse humorale spécifique contre la protéine NS3h par un test ELISA tel qu'indiqué ci-après.

Les animaux ont été sacrifiés 10 jours après la première injection et les ganglions poplités ont été prélevés pour l'analyse immunologique.

### 4. Analyses immunologiques

On a recherché la réponse humorale spécifique à la protéine NS3h précoce et localisée comme suit :
*Réponse humorale qualitative et quantitative contre la protéine NS3h -* On a effectué un prélèvement de sang sur les souris avant la première injection (J0),à J13, J27 et à J45. La présence des anticorps spécifique anti-NS3h, le titre des anticorps et les isotypes (IgG, IgG1, IgG2a) d'immunoglobulines, ont été déterminés par ELISA. Les plaques de microtitration ont été sensibilisées avec la protéine NS3h et les anticorps spécifiques contre la protéine NS3h présents dans le sérum de souris immunisées ont été révélés à l'aide de sérum de chèvre anti-IgG (H+L, Jackson Immunoresearch, Cat N° 115-035-062) de souris marqué à la peroxydase. Pour la détermination du titre des anticorps, les sérums des souris immunisés ont été dilués en série. Pour la détermination de l'isotypage, la réaction a été révélée à l'aide de sérum de chèvre anti-IgG1 de souris marqué à la peroxydase (Southern Biotechnology Associates Inc., Cat n°1070-05, Birmingham, Alabama, Usa), un anticorps de chèvre anti-IgG2a de souris marqué à la peroxydase (Interchim, UPB 90520). Le ratio des isrotypes IgG2a/IgG1, qui permet d'interpréter la tendance IFN-gamma / IL-4 (respectivement Th1-Th2) de la réponse immune, a aussi été déterminé.

### 5. Résultats

Les résultats du titre des anticorps IgG totales spécifiques anti-NS3h à Jour 30 et Jour 45 sont indiqués dans le tableau 6 ci-dessous :

**Tableau 6**

| | Titre des anticorps spécifiques anti-NS3h | |
|---|---|---|
| | Jour 30 | Jour 45 |
| NS3h | 3,3 × 10³ | 4,5 × 10⁴ |
| PLADYS/NS3h | 3,5 × 10⁴ | 2,6 × 10⁵ |
| Adjuvant de Freund/NS3h | 7,1 × 10⁴ | 1,9 × 10⁵ |

Les résulats mettent en évidence que la liaison de la protéine NS3h sur les PLA permet d'améliorer les titres en anticorps (IgG totales) d'environ 1 log par rapport au titre obtenu avec la protéine NS3h seule. Par ailleurs, les titres obtenus sont également comparables à ceux obtenus avec la formulation Adjuvant de Freund/NS3h.

L'utilisation des microparticules PLA/NS3h permet d'obtenir une réponse anticorps essentiellement IgG1 spécifique de la protéine NS3h, suggérant que la réponse est de type Th2.

### Exemple 12 : Action des PLA/NS3h au cours de la différentiation de monocytes en cellules dendritiques

Cette étude consiste à étudier l'effet sur les cellules présentatrices d'antigènes de la protéine NS3h adsorbée sur des nano-particules de PLA Pour ce faire, nous travaillons en présence de cellules dendritiques générées à l'aide de monocytes isolés à partir de sang périphérique humain et différenciés.

L'analyse de l'expansion de molécules de co-stimulation permet de dire si les cellules dendritiques immatures (DCi) au départ entrent dans un processus de maturation.

Nous testons également la capacité de l'adjuvant potentiel et de la formulation PLANS3h à favoriser la différenciation et la maturation des monocytes en cellules dendritiques. Cette étude nous permet de mieux comprendre leur rôle dans la médiation cellulaire, essentielle à l'interface entre immunité innée et adaptative. Le criblage s'effectue par étapes successives d'analyse de marqueurs phénotypiques de différenciation et de maturation et par analyse du profil de cytokines produites. L'analyse de la production de cytokines permet d'identifier si la formulation PLA-NS3 induit un profil Th1 et/ou Th2.

### 1. Purification de monocytes à partir de sang périphérique humain

Les monocytes ont été isolés à partir de sang périphérique humain sain (récupéré à l'Etablissement Français du Sang de Lyon) par centrifugation sur gradient de Ficoll et de Percoll (Arnersham Biosciences). Le Ficoll permet de créer un gradient de densité tout en conservant l'intégrité des cellules et leur fonction. Après centrifugation, les globules rouges et les polynucléaires plus denses que le Ficoll se retrouvent au fond du tube. Les PBMC (Peripheral Blood Mononuclear Cells) contenant des lymphocytes et des monocytes restent à l'interface du plasma et du Ficoll. Ceux-ci sont ensuite purifiés sur gradient de Percoll. Après centrifugation, les lymphocytes, plus denses que le Percoll, sont dans le culot tandis que les monocytes restent à l'interface du milieu et du Percoll.

Les monocytes ont été incubés avec le mélange d'anticorps (Ac) indiqués ci-après afin d'éliminer les cellules contaminantes restantes par déplétion des lymphocytes T (Ac de souris anti CD-3 OKT3 ATCC, Rockville, MD, USA dirigés contre les lymphocytes T), des lymphocytes B (Ac de souris anti CD-19 hybridome 4G7, dirigés contre les lymphocytes B), des globules rouges (Ac de souris anti Glycophorine A Immunotech) et des cellules NK (Ac de souris anti CD-56 NKH1 Immnotech dirigés contre les cellules Natural Killer) et grâce à des billes magnétiques (Dynal). Les billes Dynal sont des petites billes magnétiques recouvertes d'anticorps de mouton dirigés contre les anticorps de souris. Ces anticorps vont fixer les complexes Ac de souris/cellules puis, après passage de la suspension cellulaire sur un portoir aimanté, les cellules restantes ne seront que des monocytes.

La déplétion a été contrôlée par analyse en cytométrie de flux FACScan (Becton Dickinson).

La suspension cellulaire a été analysée par passage isolé des cellules dans une gaine liquide. Le passage de ces cellules dans un faisceau lumineux entraîne deux types de diffraction dites de petit angle (Forward scatter-FS) et de grand angle (Side scatter - SS) qui représentent les deux paramètres cellulaires pris en compte, la taille (diffraction) et la granulométrie (réfraction) des cellules. L'utilisation d'anticorps couplés à des fluorochromes (FITC : Fluorescein IsoThioCyanate lu sur l'axe des abscisses FL1 et PE : PhycoErythrine lu sur l'axe des ordonnées en FL2) excitables par le laser permet l'émission de fluorescence détectable. Un système d'amplification des signaux électriques et de convertisseur analogique digital permet la mise en forme informatique des données.

Les cellules ont été marquées à différents types d'anticorps :
- Ac anti CD-14 marqués au FTTC (spécifique des monocytes)
- Ac anti CD-3 marqués au PE (spécifique des LT)
- Ac anti CD-56 PE (des cellules NK)
- Ac anti CD-20 PE (spécifique des LB)

Le taux de contaminant est inférieur à 10%.

Les monocytes ont ensuite été mis en culture (Jour 0) dans des plaques 24 puits à raison de 1.10⁶ cellules/ml en milieu RPMI 1640 (Gibco), 2 mM de L-Glutamine (Life Technologies), 10 mM Hepès (Life Technologies), 40 ng/ml de Gentamycine (Life Technologies + 10% Sérum de Veau Foetal décomplémenté et en présence de GM-CSF (40 ng/ml) («Granular Macrophage Colony Stimulating Factor ») et d'IL-4 humaine recombinante (250U/ml). Le GM-CSF et l'IL-4 permettent la différenciation des monocytes en cellules dendritiques immatures.

### 2. Analyse phénotypique des cellules

Au 5^{ième} jour de différenciation, différents tests ont été appliqués sur les cellules :
- LPS de 250 à 2000 pg/ml → Le LPS (lipopolysaccharide) est un composant de la membrane bactérienne et est reconnu comme un signal de danger par les DCi permettant leur maturation systématiquement Une gamme de concentrations de LPS a été réalisée dans chacun des essais pour comparer les degrés de maturation.

Une dose réponse a ét effectuée pour la NS3h seule, les particules seules et les particules PLA/NS3h.
- NS3h de 1 à 50 µg/ml → témoin permettant de s'assurer que la protéine seule n'induit aucune maturation.
- PLA de 0.01 à 1 mg/ml → test permettant de voir si les PLA ont un effet adjuvant
- PLA/NS3h de 10 à 100 µl → test permettant de voir si les PLA ont un effet adjuvant en présence de la protéine NS3h.

Au 6^{ième} jour, 200 µl de surnageant ont été conservés à - 80°C pour le dosage des cytokines. Les cellules ont ensuite été collectées puis lavées.

Différents marquages témoins ont été réalisés :
- un marquage de contrôle isotypique IgG1-FTTC/ IgG2a-PE : les IgG sont capables de se fixer non spécifiquement aux cellules possédant un récepteur au fragment Fc. Ce marquage permet donc de s'assurer de la spécificité de la réaction et d'éliminer la fluorescence non spécifique des différents essais. L'utilisation d'IgG1 et d'IgG2a est liée au fait que les anticorps anti-CD utilisés sont de ce type également
- un marquage CD-14 FTTC (spécifique des monocytes) / CD-1a PE (spécifique des cellules dendritiques immatures) permet de s'assurer que la différenciation a bien fonctionné.

Le phénotype a ensuite été déterminé par marquage spécifique des cellules dendritiques matures aux :
- Ac anti CD-80 FTTC/Ac anti CD-86 PE
- Ac anti HLA-DR FTTC/Ac anti CD-83 PE
- Ac anti CD-40 PE.

### 3. Mise en oeuvre du protocole de maturation des cellules dendritiques

La mise en contact des formulations sur les cellules dendritiques immatures a été réalisée afin de juger la capacité des particules PLA/NS3h à générer des cellules dendritiques matures (effet adjuvant).

Les essais réalisés ont été les suivants :
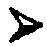 *Gamme LPS* : 250 - 300 - 400 - 500 - 750 -1000 - 2000 pg
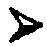 *NS3h*: 1 - 10 - 25 - 50 µg
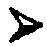 *Particules PLA* : 0.05 - 0.1 - 0.5 -1 mg
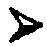 *Particules PLA*/*NS3h*: 0.24 mg DYS / 10 µg NS3h (10µl) - 0.6 mg/25 µg (25 µl) - 1.2 mg / 50 µg (50 µl) - 2.4 mg / 100 µg (100 µl).

### 4. Résultats

La protéine NS3h ne donne aucun marquage positif

Les particules de PLA DYS seules, ajoutées à J5 sur des DCi permettent d'obtenir un phénotype de maturation (DCm CD83+, CD86+, CD40+). Un effet dose-réponse sur la maturation de cellules a été observé.

Les particules PLA/NS3h donnent de très intéressants résultats. En effet, nous pouvons constater que le degré d'activation de la maturation est important Dès l'essai à 10 µl (0.24 mg PLA/10 µg hélicase), nous pouvons observer une maturation. Les PLA/NS3h ajoutés à des DCi induisent l'expression de tous les marqueurs d'activation (CD83+, CD86+, CD80+, HLA-DR+, CD40+).

L'essai à 100 µl a été réalisé tout en sachant qu'il est en large excès dans les conditions utilisées in vitro.

En conclusion, les formulations de PLA ont un effet adjuvant sur la protéine NS3h du VHC puisqu'elles permettent d'obtenir des cellules dendritiques matures.

### Exemple 13: Obtention d'anticorps monoclonaux avec les microparticules de l'invention PLA/p24

### 1. Modèle animal

Les expériences d'immunisation ont été réalisées chez des souris BALB/c (H-2^{d}) femelles âgées de 6 à 8 semaines au moment de la première immunisation.

### 2. Immunogènes administrés

Dans cette expériences, on a utilise les microparticules de l'invention PLA/p24 préparées comme indiqué dans l'exemple 1, point 2, ainsi que la composition p24-adjuvant de Freund (Sigma) préparée sous forme d'émulsion eau-dans-huile et dont il est connu qu'elle présente un bon pouvoir immunogène (témoin positif).

### 3. Immunisations

Les souris ont reçu 3 doses successives de 10 µg des immunogènes décrits dans le point 2 ci-dessus à 0, 2 et 4 semaines. Toutes les injections ont été réalisées par voie sous-cutanée. A J68 après la première injection, les réponses humorales ont été restimulées avec une injection intraveineuse de 50 µg de p24.

### 4. Suivi de l'apparition de la réponse humorale anti-p24

Afin de suivre l'apparition des anticorps anti-p24, on effectue régulièrement sur les souris des prélèvements de sang. La présence des anticorps anti-p24 est alors testée en utilisant le test ELISA similaire à celui décrit dans l'exemple 2, point 4. Cependant le conjugué de révélation est remplacé par un anticorps de chèvre anti-IgG de souris AffiniPure conjugué à la phosphatase alcaline (H+L, Jackson Immunoresearch, Cat no. 115-055-146).

### 5. Obtention d'anticorps monoclonaux

Trois jours après la dernière injection, une souris du groupe FLA-p24 a été sacrifiée ; le sang et la rate ont été prélevés. Les splénocytes obtenues à partir de la rate ont été mises en culture avec les cellules de myélome Sp2/0-Ag14 pour qu'elles fusionnent et s'immortalisent, selon le protocole décrit par Köhler et Milstein (Köhler, G. et Milstein, C., 1975, Nature, 256:495-497 ; Köhler, G. et Milstein, C., 1976, Eur. J. ImmunoL, 6:511-519). Après une période d'incubation de 12-14 jours les surnageants des hybridomes obtenus ont été criblés pour déterminer la présence d'anticorps anti-p24 en utilisant le test ELISA décrit dans le point 4 de cet exemple. Les colonies d'hybridome positives ont été sous-clonées deux fois selon la technique de la dilution limite.

### 6. Résultats

Le titre des anticorps anti-p24 dans le sérum des souris a été déterminé juste avant le sacrifice, individuellement pour chaque souris.

Les résultats sont indiqués dans le tableau 7 ci-dessous.

**Tableau 7**

| | | **Groupe PLA/p24** |
|---|---|---|
| Souris 1 | dilution 1/8000 | >3,0 (saturant) |
| | dilution 1/64000 | 0,5 |
| Souris 2 | dilution 1/8000 | >3,0 (saturant) |
| | dilution 1/64000 | 0,5 |
| Souris 3 | dilution 1/2000 | >3,0 (saturant) |
| | dilution 1/8000 | 1,3 |
| | | |

| | | **Groupe Freund/p24** |
|---|---|---|
| Souris 1 | dilution 1/8000 | >3,0 (saturant) |
| | dilution 1/64000 | 0,5 |
| Souris 2 | dilution 1/8000 | >3,0 (saturant) |
| | dilution 1/64000 | 0,5 |
| Souris 3 | dilution 1/2000 | >3,0 (saturant) |
| | dilution 1/8000 | 1,4 |

Les titres obtenus sont comparables dans les 2 groupes. Comme des anticorps monoclonaux avaient déjà été obtenus par immunisation Freund/p24, nous avons cherché à déterminer si l'immunogène PLA/p24 qui permet d'induire des titres comparables permettrait aussi d'obtenir des anticorps monoclonaux.

Pour cela, une souris du groupe PLA/p24 (souris 1) a été sacrifiée et les cellules de sa rate ont été fusionnées avec des cellules de myélome. Les hybridomes issus de la fusion ont été clonés par dilution limite dans 18 plaques 96 puits. Le criblage des surnageants de culture d'hybridome par test ELISA anti-p24 a permis d'identifier 12 clones d'hybridome qui sécrètent un anticorps spécifique de la p24. Les microparticules PLA/p24 peuvent donc être aussi utilisées pour obtenir des anticorps monoclonaux.

## Revendications

1. Procédé de préparation de microparticules biorésorbables non lamellaires sur lesquelles sont liées des substances protéiques, **caractérisé en ce qu'**il comprend les étapes de :
(i) préparation desdites microparticules par déplacement de solvant à partir d'au moins un polymère biorésorbable choisi parmi poly(acide-α-hydroxylé), poly(acide hydroxybutirique), polycaprolactones, polyorthoesters et polyanhydrides sans agent stabilisant et sans tensioactif, et
(ii) liaison desdites substances protéiques sur les microparticules obtenues dans l'étape (i) sans agent tensioactif.

2. Procédé selon la revendication 1, **caractérisé en ce que** le polymère utilisé dans l'étape (i) est un poly(acide-α-hydroxylé) ou un mélange de poly(acides-α-hydroxylés).

3. Procédé selon l'une des revendications 1 ou 2, **caractérisé en ce que** la liaison des substances protéiques sur les microparticules est mise en oeuvre par adsorption.

4. Procédé selon l'une quelconque des revendications 1 à 3, **caractérisé en ce que** la substance protéique est un antigène d'origine virale.

5. Procédé selon la revendication 4, **caractérisé en ce que** l'antigène est un antigène du virus VIH, de préférence la protéine p24 ou une protéine de régulation.

6. Procédé selon la revendication 5, **caractérisé en ce que** l'antigène du virus VIH est une protéine de régulation choisie parmi les protéines Tat, Rev et Nef.

7. Procédé selon la revendication 4, **caractérisé en ce que** l'antigène est un antigène du virus VHC, de préférence une protéine non structurale du virus VHC.

8. Procédé selon la revendication 7, **caractérisé en ce que** l'antigène est la protéine NS3, de préférence la protéine NS3 hélicase.

9. Microparticules non lamellaires biorésorbables sur lesquelles sont liées des substances protéiques, susceptibles d'être obtenues par le procédé selon d'une quelconque des revendications 1 à 8 et 17 à 20.

10. Microparticules selon la revendication 9 **caractérisée en ce que** les microparticules ont une dimension comprise entre 150 et 250 nm.

11. Utilisation des microparticules biorésorbables telles que définies dans la revendication 9 ou 10, pour la préparation d'un médicament.

12. Utilisation selon la revendication 11, **caractérisée en ce que** le médicament est destiné à l'inhibition, la prévention ou le traitement d'une infection provoquée par un virus.

13. Composition pharmaceutique, notamment vaccin, **caractérisée en ce qu'**elle contient au moins une microparticule biorésorbable telle que définie dans la revendication 9 ou 10, et le cas échéant un excipient pharmaceutiquement acceptable.

14. Composition diagnostique constituée des microparticules biorésorbables telles que définies dans la revendication 9 ou 10.

15. Utilisation de la composition diagnostique telle que définie dans la revendication 14 pour le diagnostic in vitro de l'état pathologique lié à la substance protéique liée à la microparticule biorésorbable.

16. Utilisation selon la revendication 15, **caractérisée en ce que** l'état pathologique est une infection virale.

17. Procédé selon la revendication 1, **caractérisé en ce que** le polymère de l'étape (i) est un poly(acide-α-hydroxylé) choisi parmi poly(acide-D-lactique), poly(acide-L-lactique) et poly(acide glycolique).

18. Procédé selon la revendication 1, **caractérisé en ce que** le polymère de l'étape (i) est un mélange de poly(acide-α-hydroxylé) choisi parmi un mélange de poly(acide-D et L-lactique), un mélange de poly(acide-L-lactique) et poly(acide glycolique), un mélange de poly(acide-D-lactique) et poly(acide glycolique) et un mélange de poly(acide-D et L-lactique) et poly(acide glycolique).

## Patentansprüche

1. Verfahren zur Herstellung von bioresorbierbaren nicht-lamellaren Mikropartikeln, an denen Eiweißsubstanzen gebunden sind, **dadurch gekennzeichnet, dass** es die Schritte umfasst:
(i) Herstellung der Mikropartikel durch Verdrängung eines Lösungsmittels aus mindestens einem bioresorbierbaren Polymer, das ausgewählt ist aus Poly(α-hydroxylsäure), Poly(hydroxybuttersäure), Polycaprolactone, Polyorthoester und Polyanhydride, ohne Stabilisator und ohne Tensid, und
(ii) Bindung der Eiweißsubstanzen an die in Schritt (i) erhaltenen Mikropartikel ohne Tensid.

2. Verfahren nach Anspruch 1, **dadurch gekennzeichnet, dass** das in Schritt (i) verwendete Polymer eine Poly(α-hydroxylsäure) oder eine Mischung von Poly(α-hydroxylsäuren) ist.

3. Verfahren nach einem der Ansprüche 1 oder 2, **dadurch gekennzeichnet, dass** die Bindung der Eiweißsubstanzen an den Mikropartikeln durch Adsorption durchgeführt wird.

4. Verfahren nach einem beliebigen der Ansprüche 1 bis 3, **dadurch gekennzeichnet, dass** die Eiweißsubstanz ein Antigen viralen Ursprungs ist.

5. Verfahren nach Anspruch 4, **dadurch gekennzeichnet, dass** das Antigen ein Antigen des Virus VIH, vorzugsweise p24-Protein oder Regulationsprotein, ist.

6. Verfahren nach Anspruch 5, **dadurch gekennzeichnet, dass** das Antigen des Virus VIH ein Regulationsprotein ausgewählt aus den Proteinen Tat, Rev und Nef ist.

7. Verfahren nach Anspruch 4, **dadurch gekennzeichnet, dass** das Antigen ein Antigen des Virus VHC, vorzugsweise ein nichtstrukturiertes Protein des Virus VHC ist.

8. Verfahren nach Anspruch 7, **dadurch gekennzeichnet, dass** das Antigen das Protein NS3, vorzugsweise das NS3 Helicaseprotein ist.

9. Bioresorbierbare nichtlamellare Mikropartikel, an denen Eiweißsubstanzen gebunden sind, erhältlich durch das Verfahren nach einem beliebigen der Ansprüche 1 bis 8 und 17 bis 20.

10. Mikropartikel nach Anspruch 9, **dadurch gekennzeichnet, dass** die Mikropartikel eine Dimension zwischen 150 und 250 nm aufweisen.

11. Verwendung von bioresorbierbaren Mikropartikeln, wie sie in den Ansprüchen 9 oder 10 definiert sind, für die Herstellung eines Medikaments.

12. Verwendung nach Anspruch 11, **dadurch gekennzeichnet, dass** das Medikament zur Hemmung, der Prävention oder der Behandlung einer Infektion, die durch einen Virus hervorgerufen wird, dient.

13. Pharmazeutische Zusammensetzung, insbesondere Impfstoff, **dadurch gekennzeichnet, dass** sie mindestens ein bioresorbierbares Mikropartikel, wie es in dem Anspruch 9 oder 10 definiert ist, und gegebenenfalls einen pharmazeutisch annehmbaren Hilfsstoff enthält.

14. Diagnostische Zusammensetzung, bestehend aus den bioresorbierbaren Mikropartikeln, wie im Anspruch 9 oder 10 definiert.

15. Verwendung der diagnostischen Zusammensetzung, wie sie im Anspruch 14 definiert ist, für die in vitro-Diagnose des pathologischen Zustands gebunden an die Eiweißsubstanz, die an das bioresorbierbare Mikropartikel gebunden ist.

16. Verwendung nach Anspruch 15, **dadurch gekennzeichnet, dass** der pathologische Zustand eine Virusinfektion ist.

17. Verfahren nach Anspruch 1, **dadurch gekennzeichnet, dass** das Polymer des Schritts (i) eine Poly(α-hydroxylsäure) ausgewählt aus Poly(D-Milchsäure), Poly(L-Milchsäure) und Poly(glycolsäure), ist.

18. Verfahren nach Anspruch 1, **dadurch gekennzeichnet, dass** das Polymer des Schritts (i) eine Poly(α-hydroxylsäure) Mischung ist, ausgewählt aus einer Mischung von Poly(D- und L-Milchsäure), einer Mischung aus Poly(L-Milchsäure) und Poly(glycolsäure), einer Mischung aus Poly(D-Milchsäure) und Poly(glycolsäure) und einer Mischung aus Poly(D- und L-Milchsäure) und Poly(glycolsäure).

## Claims

1. Method for the preparation of bioresorbable non-lamellar microparticles to which protein substances are bonded, **characterised in that** it comprises the steps of:
i. preparing said microparticles by solvent displacement starting from at least one bioresorbable polymer selected from the group consisting of poly(α-hydroxy acids), poly(hydroxybutyric acids), polycaprolactones, polyorthoesters and polyanhydrides without stabilising agent and without surfactant, and
ii. bonding said protein substances to the microparticles obtained in step (i) without surfactant.

2. Method according to claim 1, **characterised in that** the polymer used in step (i) is a poly(α-hydroxy acid) or a mixture of poly(α-hydroxy acids).

3. Method according to one of claims 1 or 2, **characterised in that** the bonding of the protein substances to the microparticles is conducted by adsorption.

4. Method according to any one of claims 1 to 3, **characterised in that** the protein substance is an antigen of viral origin.

5. Method according to claim 4, **characterised in that** the antigen is an antigen of the HIV virus, preferably the p24 protein or a regulatory protein.

6. Method according to claim 5, **characterised in that** the antigen of the HIV virus is a regulatory protein selected from the Tat, Rev and Nef proteins.

7. Method according to claim 4, **characterised in that** the antigen is an antigen of the CHV virus, preferably a non-structural protein of the CHV virus.

8. Method according to claim 7, **characterised in that** the antigen is the NS3 protein, preferably the helicase NS3 protein.

9. Bioresorbable non-lamellar microparticles to which protein substances are bonded that can be obtained by the method according to any one of claims 1 to 8 and 17 to 20.

10. Microparticles according to claim 9, **characterised in that** the particles have a dimension in the range of between 150 and 250 nm.

11. Use of the bioresorbable microparticles as defined in claim 9 or 10 for the preparation of a medication.

12. Use according to claim 11, **characterised in that** the medication is intended for the inhibition, prevention or treatment of an infection caused by a virus.

13. Pharmaceutical composition, in particular vaccine, **characterised in that** it contains at least one bioresorbable microparticle as defined in claim 9 or 10 and, if applicable, a pharmaceutically acceptable excipient.

14. Diagnostic composition formed from the bioresorbable microparticles as defined in claim 9 or 10.

15. Use of the diagnostic composition as defined in claim 14 for the in vitro diagnosis of the pathological condition linked to the protein substance bonded to the bioresorbable microparticle.

16. Use according to claim 15, **characterised in that** the pathological condition is a viral infection.

17. Method according to claim 1, **characterised in that** the polymer of step (i) is a poly(α-hyroxy acid) selected from poly(D-lactic acid), poly(L-lactic acid) and poly(glycolic acid).

18. Method according to claim 1, **characterised in that** the polymer of step (i) is a mixture of poly(α-hyroxy acid) selected from a mixture of poly(D-lactic acid) and poly(L-lactic acid), a mixture of poly(L-lactic acid) and poly(glycolic acid), a mixture of poly(D-lactic acid) and poly(glycolic acid) and a mixture of poly(D- and L-lactic acid) and poly(glycolic acid).


### SEQUENCE LISTING

<110> bioMérieux
<120> Procédé de préparation de microparticules biorésorbables, microparticules obtenues et utilisation
<130> Palprot
<160> 32
<170> PatentIn version 3.1
<210> 1
   <211> 101
   <212> PRT
   <213> HIV
<400> 1
   Met Glu Pro Val Asp Pro Arg Leu Glu Pro Trp Lys His Pro Gly Ser
   1               5                   10                  15
   Gln Pro Arg Thr Ala Cys Thr Asn Cys Tyr Cys Lys Lys Cys Cys Phe
               20                  25                  30
   His Cys Gln Val Cys Phe Ile Arg Lys Ala Leu Gly Ile Ser Tyr Gly
           35                  40                  45
   Arg Lys Lys Arg Arg Gln Arg Arg Arg Ala Pro Gln Asp Ser Glu Thr
       50                  55                  60
   His Gln Val Ser Pro Pro Lys Gln Pro Ala Ser Gln Pro Arg Gly Asp
   65                  70                  75                  80
   Pro Thr Gly Pro Lys Glu Ser Lys Lys Lys Val Glu Arg Glu Thr Glu
                   85                  90                  95
   Thr His Pro Val Asn
               100
<210> 2
   <211> 288
   <212> PRT
   <213> HCV
<400> 2
   Met Arg Gly Ser His His His His His His Gly Ser Val Asp Glu Ser
   1               5                   10                  15
   Met Asp Glu Phe Ala Val Asp Phe Ile Pro Val Glu Asn Leu Glu Thr
               20                  25                  30
   Thr Met Arg Ser Pro Val Phe Ser Asp Asn Ser Ser Pro Pro Ala Val
           35                  40                  45
   Pro Gln Ser Tyr Gln Val Ala His Leu His Ala Pro Thr Gly Ser Gly
       50                  55                  60
   Lys Ser Thr Lys Val Pro Ala Ala Tyr Ala Ala Gln Gly Tyr Lys Val
   65 70 75 80
   Leu Val Leu Asn Pro Ser Val Ala Ala Thr Leu Gly Phe Gly Ala Tyr
   85 90 95
   Met Ser Lys Ala His Gly Ile Asp Pro Asn Ile Arg Thr Gly Val Arg
   100 105 110
   Thr Ile Thr Thr Gly Ser Pro Ile Thr Tyr Ser Thr Tyr Gly Lys Phe
   115 120 125
   Leu Ala Asp Gly Gly Cys Ser Gly Gly Ala Tyr Asp Ile Ile Ile Cys
   130 135 140
   Asp Glu Cys His Ser Thr Asp Ala Thr Ser Ile Leu Gly Ile Gly Thr
   145 150 155 160
   Val Leu Asp Gln Ala Glu Thr Ala Gly Ala Arg Leu Thr Val Leu Ala
   165 170 175
   Thr Ala Thr Pro Pro Gly Ser Val Thr Val Pro His Pro Asn Ile Glu
   180 185 190
   Glu Val Ala Leu Ser Thr Thr Gly Glu Ile Pro Phe Tyr Gly Lys Ala
   195 200 205
   Ile Pro Leu Glu Ala Ile Lys Gly Gly Arg His Leu Ile Phe Cys His
   210 215 220
   Ser Lys Lys Lys Cys Asn Glu Leu Ala Ala Lys Leu Val Ala Leu Gly
   225 230 235 240
   Val Asn Ala Val Ala Tyr Tyr Arg Gly Leu Asp Val Ser Val Ile Pro
   245 250 255
   Thr Ser Gly Asp Val Val Val Val Ala Thr Asp Ala Leu Met Thr Gly
   260 265 270
   Phe Thr Gly Asp Phe Asp Ser Val Ile Asp Cys Asn Thr Cys Val Ile
   275 280 285
<210> 3
   <211> 9
   <212> PRT
   <213> HIV
<400> 3
   Ala Met Gln Met Leu Lys Glu Thr Ile
   1 5
<210> 4
   <211> 15
   <212> PRT
   <213> HIV
<400> 4
   Cys Phe His Cys Gln Val Cys Phe Thr Lys Lys Gly Leu Gly Ile
   1 5 10 15
<210> 5
   <211> 15
   <212> PRT
   <213> HIV
<400> 5
   Val Cys Phe Thr Lys Lys Gly Leu Gly Ile Ser Tyr Gly Arg Lys
   1 5 10 15
<210> 6
   <211> 15
   <212> PRT
   <213> HIV
<400> 6
   Lys Gly Leu Gly Ile Ser Tyr Gly Arg Lys Lys Arg Arg Gln Arg
   1 5 10 15
<210> 7
   <211> 15
   <212> PRT
   <213> HIV
<400> 7
   Ser Tyr Gly Arg Lys Lys Arg Arg Gln Arg Arg Arg Ser Pro Gln
   1 5 10 15
<210> 8
   <211> 15
   <212> PRT
   <213> HIV
<400> 8
   Lys Arg Arg Gln Arg Arg Arg Ser Pro Gln Asp Ser Glu Thr His
   1 5 10 15
<210> 9
   <211> 15
   <212> PRT
   <213> HIV
<400> 9
   Arg Arg Ser Pro Gln Asp Ser Glu Thr His Gln Val Ser Leu Ser
   1 5 10 15
<210> 10
   <211> 2838
   <212> DNA
   <213> HCV
<400> 10
   gcgcctatca cggcctattc ccaacaaacg cggggcctgc ttggctgtat catcactagc      60
   ctcacaggtc gggacaagaa ccaggtcgat ggggaggttc aggtgctctc caccgcaacg     120
   caatctttcc tggcgacctg cgtcaatggc gtgtgttgga ccgtctacca tggtgccggc     180
   tcgaagaccc tggccggccc gaagggtcca atcacccaaa tgtacaccaa tgtagaccag     240
   gacctcgtcg gctggccggc gccccccggg gcgcgctcca tgacaccgtg cacctgcggc     300
   agctcggacc tttacttggt cacgaggcat gccgatgtca ttccggtgcg ccggcgaggc     360
   gacagcaggg ggagtctact ctcccctagg cccgtctcct acctgaaggg ctcctcgggt     420
   ggaccactgc tttgcccttc ggggcacgtt gtaggcatct tccgggctgc tgtgtgcacc     480
   cggggggttg cgaaggcggt ggacttcata cccgttgagt ctatggaaac taccatgcgg     540
   tctccggtct tcacagacaa ctcatcccct ccggccgtac cgcaaacatt ccaagtggca     600
   catttacacg ctcccactgg cagcggcaag agcaccaaag tgccggctgc atatgcagcc     660
   caagggtaca aggtgcgcgt cctaaacccg tccgttgctg ccacattggg ctttggagcg     720
   tatatgtcca aggcacatgg catcgagcct aacatcagaa ctggggtaag gaccatcacc     780
   acgggcggcc ccatcacgta ctccacctat ggcaagttcc ttgccgacgg tggatgctcc     840
   gggggcgcct atgacatcat aatatgtgac gaatgccact caactgactg gacaaccatc     900
   ttgggcatcg gcacagtcct ggatcaggca gagacggctg gagcgcggct cgtcgtgctc     960
   gccaccgcca cgcctccggg atcgatcacc gtgccacacc ccaacatcga ggaagtggcc    1020
   ctgtccaaca ctggggagat tcccttctat ggcaaagcca tccccattga ggccatcaag    1080
   gggggaaggc atctcatctt ctgccattcc aagaagaagt gtgacgagct cgccgcaaag    1140
   ctgacaggcc tcggactcaa cgctgtagcg tattacaggg gtctcgatgt gtccgtcata    1200
   ccgactagcg gagacgtcgt tgtcgtggca acagacgctc taatgacggg ctttaccggc    1260
   gactttgact cagtgatcga ctgcaacaca tgtgtcaccc agacagtcga tttcagcttg    1320
   gatcccacct tcaccattga gacgaccacc gtgccccaag acgcggtgtc gcgctcgcag    1380
   cggcgaggta ggactggcag gggcaggagt ggcatctaca ggtttgtgac tccaggagaa    1440
   cggccctcag gcatgttcga ctcctcggtc ctgtgtgagt gctatgacgc aggctgcgct    1500
   tggtatgagc tcacgcccgc tgagactaca gtcaggttgc gggcttacct gaatacacca    1560
   gggttgcccg tctgccagga ccatctggag ttctgggaaa gcgtcttcac aggcctcacc    1620
   cacatagatg cccacttcct gtcccaaacc aagcaggcag gagacaactt cccctacctg    1680
   gtggcatacc aagccacggt gtgcgccagg gctcaggctc cacctccatc gtgggatcaa 1740
   atgtggaagt gtctcatacg gcttaaacct acgctgcacg ggccaacacc cctgctgtat 1800
   aggctaggag ccgttcaaaa tgagatcacc ctcacacatc ccataaccaa attcgtcatg 1860
   gcatgcatgt cggccgacct ggaggtcgtc actagcacct gggtgctggt aggcggagtc 1920
   cttgcagctc tggccgcata ttgcctgaca accggtagtg tggtcattgt gggtaggatc 1980
   attttgtccg ggaggccggc tgttgttccc gacagggaag tcctctaccg ggagttcgat 2040
   gaaatggaag agtgcgcctc acacctccct tacatcgagc aaggaatgca gctcgccgag 2100
   cagttcaagc agaaggcact cgggttgctg caaacagcca ccaagcaagc ggaggccgct 2160
   gctcccgtgg tggagtccag gtggcgggcc cttgaggcct tctgggcaaa gcacatgtgg 2220
   aacttcatca ccgggataca gtacttagca ggcttatcca ctctgcctgg gaaccccgcg 2280
   atagcatcac tgatggcatt cacagcctct atcaccagtc cgctcaccac ccagaatacc 2340
   ctcctattca acatcttagg gggatgggtg gctgctcaac tcgctcctcc cagtgctgct 2400
   tcggccttcg tgggtgccgg cattgccggt gcggccattg gcagcatagg ccttgggaag 2460
   gtgcttgtgg acattctggc gggctatgga gcgggggtgg ccggtgcact cgtggctttt 2520
   aaggtcatga gcggcgaggc gccctccgcc gaggacctgg ttaacttgct ccctgccatc 2580
   ctctcccccg gcgccttggt cgtcgggatc gtgtgtgcag caatcctgcg tcggcacgtg 2640
   ggcccgggag agggggctgt gcagtggatg aaccggctga tagcgttcgc ttcgcggggt 2700
   aaccacgttt cccccacgca ctacgtgcct gagagcgacg ccgcagcacg tgtaactcag 2760
   atcctctcca gcctcaccat cactcagctg ctgaagaggc ttcaccagtg gattaatgag 2820
   gactgctcca cgccatgc 2838
<210> 11
   <211> 10
   <212> PRT
   <213> HCV
<400> 11
   Lys Leu Val Ala Leu Gly Val Asn Ala Val
   1 5 10
<210> 12
   <211> 15
   <212> PRT
   <213> HIV
<400> 12
   Glu Gln Ile Gly Trp Met Thr Asn Asn Pro Pro Ile Pro Val Gly
   1 5 10 15
<210> 13
   <211> 15
   <212> PRT
   <213> HIV
<400> 13
   Trp Met Thr Asn Asn Pro Pro Ile Pro Val Gly Glu Ile Tyr Lys
   1 5 10 15
<210> 14
   <211> 15
   <212> PRT
   <213> HIV
<400> 14
   Asn Pro Pro Ile Pro Val Gly Glu Ile Tyr Lys Arg Trp Ile Ile
   1 5 10 15
<210> 15
   <211> 15
   <212> PRT
   <213> HIV
<400> 15
   Pro Val Gly Glu Ile Tyr Lys Arg Trp Ile Ile Leu Gly Leu Asn
   1 5 10 15
<210> 16
   <211> 15
   <212> PRT
   <213> HIV
<400> 16
   Ile Tyr Lys Arg Trp Ile Ile Leu Gly Leu Asn Lys Ile Val Arg
   1 5 10 15
<210> 17
   <211> 15
   <212> PRT
   <213> HIV
<400> 17
   Trp Ile Ile Leu Gly Leu Asn Lys Ile Val Arg Met Tyr Ser Pro
   1 5 10 15
<210> 18
   <211> 15
   <212> PRT
   <213> HIV
<400> 18
   Gly Leu Asn Lys Ile Val Arg Met Tyr Ser Pro Thr Ser Ile Leu
   1 5 10 15
<210> 19
   <211> 15
   <212> PRT
   <213> HIV
<400> 19
   Ile Val Arg Met Tyr Ser Pro Thr Ser Ile Leu Asp Ile Arg Gln
   1 5 10 15
<210> 20
   <211> 15
   <212> PRT
   <213> HIV
<400> 20
   Tyr Ser Pro Thr Ser Ile Leu Asp Ile Arg Gln Gly Pro Lys Glu
   1 5 10 15
<210> 21
   <211> 15
   <212> PRT
   <213> HIV
<400> 21
   Ser Ile Leu Asp Ile Arg Gln Gly Pro Lys Glu Pro Phe Arg Asp
   1 5 10 15
<210> 22
   <211> 15
   <212> PRT
   <213> HIV
<400> 22
   Ile Arg Gln Gly Pro Lys Glu Pro Phe Arg Asp Tyr Val Asp Arg
   1 5 10 15
<210> 23
   <211> 15
   <212> PRT
   <213> HIV
<400> 23
   Pro Lys Glu Pro Phe Arg Asp Tyr Val Asp Arg Phe Tyr Lys Thr
   1 5 10 15
<210> 24
   <211> 15
   <212> PRT
   <213> HIV
<400> 24
   Phe Arg Asp Tyr Val Asp Arg Phe Tyr Lys Thr Leu Arg Ala Glu
   1 5 10 15
<210> 25
   <211> 15
   <212> PRT
   <213> HIV
<400> 25
   Val Asp Arg Phe Tyr Lys Thr Leu Arg Ala Glu Gln Ala Ser Gln
   1 5 10 15
<210> 26
   <211> 15
   <212> PRT
   <213> HIV
<400> 26
   Tyr Lys Thr Leu Arg Ala Glu Gln Ala Ser Gln Glu Val Lys Asn
   1 5 10 15
<210> 27
   <211> 15
   <212> PRT
   <213> HIV
<400> 27
   Arg Ala Glu Gln Ala Ser Gln Glu Val Lys Asn Trp Met Thr Glu
   1 5 10 15
<210> 28
   <211> 15
   <212> PRT
   <213> HIV
<400> 28
   Ala Ser Gln Glu Val Lys Asn Trp Met Thr Glu Thr Leu Leu Val
   1 5 10 15
<210> 29
   <211> 15
   <212> PRT
   <213> HIV
<400> 29
   Val Lys Asn Trp Met Thr Glu Thr Leu Leu Val Gln Asn Ala Asn
   1 5 10 15
<210> 30
   <211> 15
   <212> PRT
   <213> HIV
<400> 30
   Met Thr Glu Thr Leu Leu Val Gln Asn Ala Asn Pro Asp Cys Lys
   1 5 10 15
<210> 31
   <211> 15
   <212> PRT
   <213> HIV
<400> 31
   Leu Leu Val Gln Asn Ala Asn Pro Asp Cys Lys Thr Ile Leu Lys
   1 5 10 15
<210> 32
   <211> 15
   <212> PRT
   <213> HIV
<400> 32
   Asn Ala Asn Pro Asp Cys Lys Thr Ile Leu Lys Ala Leu Gly Pro
   1 5 10 15